# EUROPEAN PATENT APPLICATION

(11) **EP 4 527 315 A1**
(43) Date of publication of application: **26.03.2025**
(21) Application number: 23831704.4
(22) Date of filing: 02.05.2023
(51) Int. Cl.: A61B 10/00, A61B 5/00, A61B 5/0205, A61B 5/024, A61B 5/01

(54) **WEARABLE DEVICE, METHOD, AND COMPUTER-READABLE STORAGE MEDIUM FOR PROVIDING SERVICE RELATED TO USER HEALTH**

(30) Priority: 26.06.2022 KR 20220077910; 30.08.2022 KR 20220109517
(71) Applicant: Samsung Electronics Co., Ltd., Suwon-si, Gyeonggi-do 16677 (KR)
(72) Inventor: LEE, Hongji, Suwon-si Gyeonggi-do 16677 (KR); HWANG, Minkyung, Suwon-si Gyeonggi-do 16677 (KR)
(74) Representative: Appleyard Lees IP LLP
(86) International application number: PCT/KR2023/005986
(87) International publication number: WO 2024/005340

(57) **Abstract**

According to an embodiment, a wearable device comprises a plurality of sensors and a processor. The processor is configured to: identify a heart rate of a user on the basis of a signal acquired via a portion of a heart rate sensor, among the plurality of sensors, which is in contact with a first portion of a user's body; determine that the identified heart rate is equal to or higher than a reference heart rate; provide, in response to determining that the identified heart rate is equal to or higher than the reference heart rate, a first guide that proposes contacting a portion of a body temperature sensor, among the plurality of sensors, with a designated second portion of the body; acquire data regarding a body temperature of the user via the body temperature sensor after the first guide is provided; and output a notification related to the measurement of the body temperature at least partially on the basis of the acquisition of the data regarding the body temperature.

## Description

### [Technical Field]

The following descriptions relate to a wearable device, a method, and a computer-readable storage media for providing a service related to a user's health.

### [Background Art]

Various women's health services are provided through a wearable device. A women's health service may include a service that helps when planning for pregnancy or contraception by providing a menstrual cycle, a fertile window (or ovulation day), and/or a contraceptive period of a woman (or user). The wearable device may identify information on a female user and provide the women's health service based on the identified information.

### [Disclosure]

### [Technical Problem]

Various diseases (or illness) may occur to a user (or female user) of a wearable device. Even when at least one symptom according to the various diseases occurs, the user may not recognize it.

The technical problems to be achieved in this document are not limited to those described above, and other technical problems not mentioned herein will be clearly understood by those having ordinary knowledge in the art to which the present disclosure belongs, from the following description.

### [Technical Solution]

According to an embodiment, a wearable device may comprise a plurality of sensors including a heart rate sensor, a motion sensor, and a body temperature sensor, memory including a plurality of program instructions, and a processor operably coupled with the plurality of sensors and the memory, wherein the program instructions, when executed by the processor, may cause the wearable device to identify, based on a signal obtained via a portion of the heart rate sensor contacted with a first portion of a body of a user, a heart rate of the user, determine that the identified heart rate is greater than or equal to a reference heart rate, provide, in response to determining that the identified heart rate is greater than or equal to the reference heart rate, a first guide proposing to contact a portion of the body temperature sensor on a second portion of the body which is designated, after the first guide is provided, obtain, via the body temperature sensor, data related to a body temperature of the user, and output, based at least in part on obtaining the data related to the body temperature, notification related to measurement of the body temperature.

According to an embodiment, a method of a wearable device may comprise identifying, based on a signal obtained via a portion of a heart rate sensor of the wearable device contacted with a first portion of a body of a user, a heart rate of the user, determining that the identified heart rate is greater than or equal to a reference heart rate, providing, in response to determining that the identified heart rate is greater than or equal to the reference heart rate, a first guide proposing to contact a portion of a body temperature sensor of the wearable device with a second portion of the body which is designated, after the first guide is provided, obtaining, via the body temperature sensor, data related to a body temperature of the user, and outputting, based at least in part on obtaining the data related to the body temperature, notification related to measurement of the body temperature.

According to an embodiment, one or more programs stored in a non-transitory computer readable storage medium may comprise instructions which, when executed by a processor of a wearable device with a heart rate sensor, a body temperature sensor, and a motion sensor, cause the wearable device to identify, based on a signal obtained via a portion of the heart rate sensor contacted with a first portion of a body of a user, a heart rate of the user, determine that the identified heart rate is greater than or equal to a reference heart rate, provide, in response to determining that the identified heart rate is greater than or equal to the reference heart rate, a first guide proposing to contact a portion of the body temperature sensor on a second portion of the body which is designated, after the first guide is provided, obtain, via the body temperature sensor, data related to a body temperature of the user, and output, based at least in part on obtaining the data related to the body temperature, notification related to measurement of the body temperature.

### [Advantageous Effects]

According to an embodiment, a wearable device can identify a user's biometric information by using at least one sensor. The wearable device can provide a service for a woman's health by using the identified user's biometric information.

The effects that can be obtained from the present disclosure are not limited to those described above, and any other effects not mentioned herein will be clearly understood by those having ordinary knowledge in the art to which the present disclosure belongs, from the following description.

### [Description of the Drawings]

FIG. 1 is a block diagram of an electronic device in a network environment according to an embodiment.
FIGS. 2A and 2B illustrate perspective views of an exemplary electronic device according to an embodiment.
FIG. 3 illustrates an exploded perspective view of an exemplary electronic device according to an embodiment.
FIG. 4 is a simplified block diagram of a wearable device according to an embodiment.
FIG. 5 is a flowchart illustrating an exemplary operation of a wearable device according to an embodiment.
FIG. 6A is a flowchart illustrating an exemplary operation of a wearable device for identifying a first symptom according to an embodiment.
FIG. 6B is a flowchart illustrating an exemplary operation of a wearable device for setting a lock state or an unlock state according to an embodiment.
FIG. 7 illustrates an exemplary operation of a wearable device for identifying a first symptom according to an embodiment.
FIG. 8 is a flowchart illustrating an exemplary operation of a wearable device for identifying a second symptom according to an embodiment.
FIG. 9 is a flowchart illustrating an example of an operation of a wearable device for identifying a third symptom according to an embodiment.
FIG. 10 is a flowchart illustrating an exemplary operation of a wearable device for identifying a fourth symptom according to an embodiment.
FIG. 11 is a flowchart illustrating an exemplary operation of a wearable device for identifying a fifth symptom according to an embodiment.
FIG. 12 is a flowchart illustrating an exemplary operation of a wearable device for identifying a sixth symptom according to an embodiment.
FIG. 13 illustrates an example of patterns related to a change in body temperature of a user according to an embodiment.
FIG. 14 is a flowchart illustrating an exemplary operation of a wearable device for identifying a seventh symptom according to an embodiment.
FIG. 15 illustrates an example of patterns related to a change in body temperature of a user according to an embodiment.
FIG. 16 is a flowchart illustrating an exemplary operation of a wearable device for displaying information on at least one symptom occurred to a user according to an embodiment.
FIGS. 17A and 17B illustrate an example of an operation of an electronic device according to an embodiment.
FIGS. 18A and 18B illustrate an example of an operation of an electronic device according to an embodiment.
FIG. 19 illustrates an example of an operation of an electronic device according to an embodiment.
FIG. 20 is a flowchart illustrating an exemplary operation of a wearable device for displaying information on at least one symptom occurred to a user according to an embodiment.
FIG. 21 illustrates an example of an operation of an electronic device according to an embodiment.
FIG. 22 illustrates an example of an operation of an electronic device according to an embodiment.
FIG. 23 is a flowchart illustrating an exemplary operation of a wearable device for identifying a step indicating a health state of a user according to an embodiment.
FIGS. 24A and 24B illustrate an example of an operation of an electronic device according to an embodiment.

### [Mode for Invention]

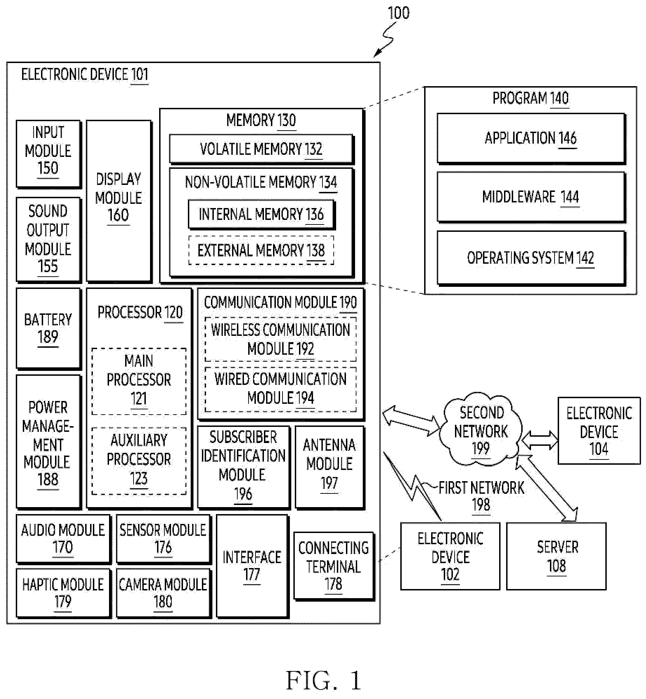

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

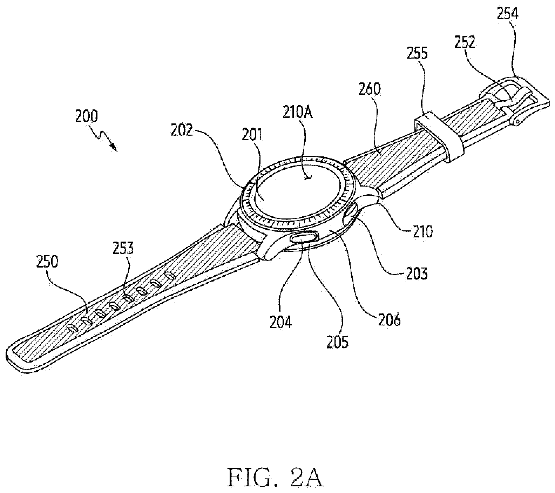

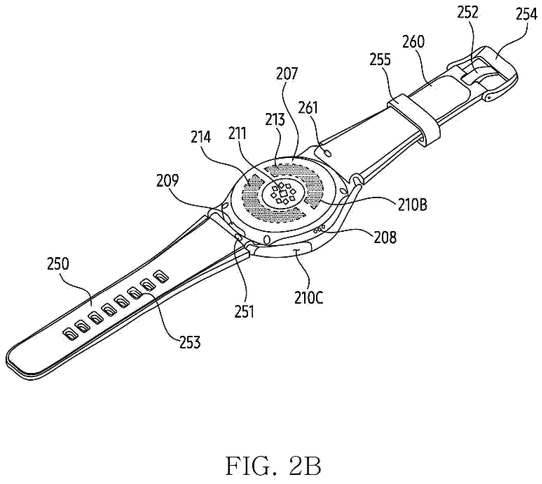

FIGS. 2A and 2B are perspective views of an electronic device according to an embodiment.

Referring to FIGS. 2A and 2B, an electronic device 200 (e.g., the electronic device 101 of FIG. 1) according to an embodiment may include a housing 210 including a first surface (or front surface) 210A, a second surface (or rear surface) 210B, and a side surface 210C surrounding the space between the first surface 210A and the second surface 210B and binding members 250 and 260 connected to at least a part of the housing 210 and configured to detachably attach the electronic device 200 to a part of the user's body (e.g., wrist, ankle, etc.). In another embodiment (not illustrated), the housing may also refer to a structure that forms at least a part of the first surface 210A, the second surface 210B, and the side surface 210C of FIG. 2A. According to an embodiment, at least a part of the first surface 210A may be implemented by a substantially transparent front plate 201 (e.g., glass plate or polymer plate including various coating layers). The second surface 210B may be implemented by a substantially opaque rear plate 207. The rear plate 207 may be made of, for example, coated or colored glass, ceramic, polymer, metal (e.g., aluminum, stainless steel (STS), or magnesium), or a combination of at least two of the materials. The side surface 210C may be coupled to the front plate 201 and the rear plate 207 and may be implemented by a side bezel structure (or "side member") 206 including metal and/or polymer. In some embodiments, the rear plate 207 and the side bezel structure 206 may be integrated together and may include the same material (e.g., metal material such as aluminum). The binding members 250 and 260 may be made of various materials and may be made in various shapes. The binding members 250 and 260 may be made of woven fabric, leather, rubber, urethane, metal, ceramic, or a combination of at least two of the materials.

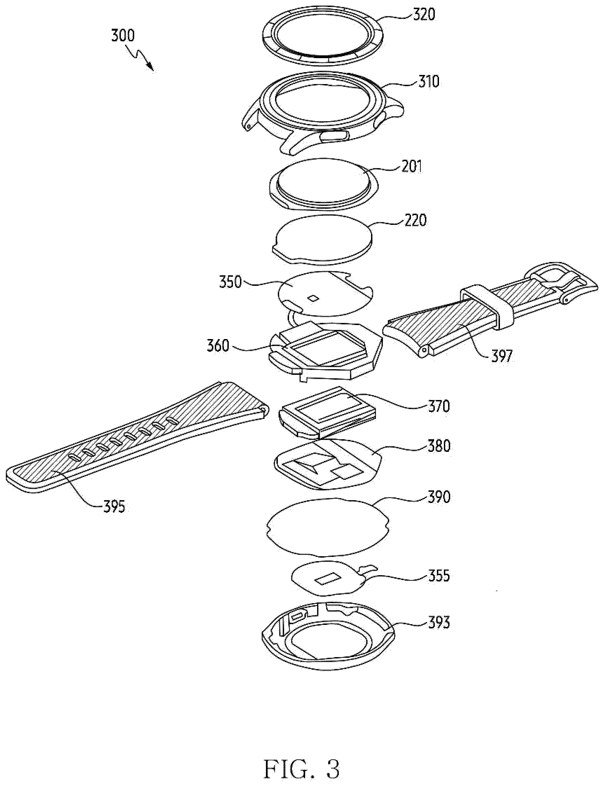

According to an embodiment, the electronic device 200 may include at least one of a display 220 (see FIG. 3), an audio module 205 and 208, a sensor module 211, a key input device 202, 203 and 204, and a connector hole 209. In some embodiments, the electronic device 200 may omit at least one of the components (e.g., the key input devices 202, 203 and 204, the connector hole 209, or the sensor module 211) or may further include another component.

The display 220 may be exposed, for example, through a substantial portion of the front plate 201. The shape of the display 220 may correspond to the shape of the front plate 20, such as circular (shown in FIG. 2), oval, or polygonal. The display 220 may be coupled to or adjacent to a touch sensing circuit, a pressure sensor capable of measuring the strength (pressure) of touches, and/or a fingerprint sensor.

The audio modules 205 and 208 may include a microphone hole 205 and a speaker hole 208. Corresponding to the microphone hole 205, a microphone for obtaining external sound may be disposed inside, and in some embodiments, a plurality of microphones may be disposed to detect the direction of the sound. The speaker hole 208 may be used with an external speaker and a receiver for phone calls. In some embodiments, the speaker hole 208 and the microphone hole 205 may be implemented as a single hole, or a speaker (e.g., piezo speaker) may be included without the speaker hole 208.

The sensor module 211 may generate electrical signal(s) or data value(s) corresponding to internal operating state(s) of the electronic device 200 or external environmental state(s). The sensor module 211 may include, for example, a biometric sensor module 211 (e.g., heart-rate monitor (HRM) sensor) disposed on the second surface 210B of the housing 210. The electronic device 200 may further include at least one sensor module not shown, such as a gesture sensor, a gyro sensor, a pressure sensor, a magnetic sensor, an acceleration sensor, a grip sensor, a color sensor, an infrared sensor, a biometric sensor, a humidity sensor, and/or an illumination sensor.

The sensor module 211 may include electrode regions 213 and 214 forming a part of the surface of the electronic device 200 and a bio-signal detection circuit (not shown) electrically connected to the electrode regions 213 and 214. For example, the electrode regions 213 and 214 may include the first electrode region 213 and the second electrode region 214 disposed on the second surface 210B of the housing 210. The sensor module 211 may be configured such that the electrode regions 213 and 214 obtain electrical signal(s) from a part of the user's body, and the bio-signal detection circuit may detect biometric information of the user based on the electrical signal(s).

The key input devices 202, 203, and 204 may include a wheel key 202 disposed on the first surface 210A of the housing 210 and rotatable in at least one direction, and/or side key buttons 203 and 204 disposed on the side surface 210C of the housing 210. The wheel key may have a shape corresponding to the shape of the front plate 201. In another embodiment, the electronic device 200 may not include some or all of the above-described key input devices 202, 203, and 204, and the not included key input devices 202, 203, and 204 may be implemented in other forms such as soft keys on the display 220. The connector hole 209 may accommodate a connector (e.g., USB connector) for transmitting and receiving power and/or data to and from external electronic devices and may include another connector hole (not illustrated) capable of accommodating a connector for transmitting and receiving audio signals to and from an external electronic device. The electronic device 200 may further include, for example, a connector cover (not illustrated) that covers at least a part of the connector hole 209 and blocks the inflow of external foreign material into the connector hole.

The binding members 250 and 260 may be detachably attached to at least a part of the housing 210 using locking members 251, 261. The binding members 250 and 260 may include one or more of a fixing member 252, a fixing member fastening hole 253, a band guide member 254, and a band fixing ring 255.

The fixing member 252 may be configured to fix the housing 210 and the binding members 250 and 260 to a part of the user's body (e.g., wrist, ankle, etc.). The fixing member fastening hole 253 may correspond to the fixing member 252 to fix the housing 210 and the binding members 250 and 260 to the part of the user's body. The band guide member 254 may be configured to limit movement range of the fixing member 252 when the fixing member 252 is fastened to the fixing member fastening hole 253, so that the binding members 250 and 260 are attached to be in close contact with the part of the user's body. The band fixing ring 255 may limit the range of movement of the fixing members 250 and 260 when the fixing member 252 and the fixing member fastening hole 253 are fastened.

FIG. 3 is an exploded perspective view of an electronic device according to an embodiment.

Referring to FIG. 3, an electronic device 300 (e.g., the electronic device 101 of FIG. 1, the electronic device 200 of FIG. 2A and/or FIG. 2B) may include a side bezel structure 310, a wheel key 320, a front plate 201, a display 220, a first antenna 350, a second antenna 355, a support member 360 (e.g., bracket), a battery 370, a printed circuit board 380, a sealing member 390, a rear plate 393, and binding members 395 and 397. At least one of the components of the electronic device 300 may be the same as or similar to at least one of the components of the electronic device 200 of FIGS. 2A, and/or 2B, and repeated description thereof will be omitted. The support member 360 may be disposed inside the electronic device 300 to be connected to the side bezel structure 310 or may be integrated with the side bezel structure 310. The support member 360 may be made of, for example, metal material and/or non-metal (e.g., polymer) material. With respect to the support member 360, the display 220 may be coupled to one surface and the printed circuit board 380 may be coupled to the other surface. A processor, a memory, and/or an interface may be mounted on the printed circuit board 380. The processor may include, for example, one or more of a central processing unit, an application processor, a graphic processing unit (GPU), an application processor sensor processor, or a communication processor.

The memory may include, for example, a volatile memory or a nonvolatile memory. The interface may include, for example, a high-definition multimedia interface (HDMI), a universal serial bus (USB) interface, an SD card interface, and/or an audio interface. The interface may electrically or physically connect the electronic device 300 to an external electronic device, for example, and may include a USB connector, an SD card/MMC connector, or an audio connector.

The battery 370 is a device for supplying power to at least one component of the electronic device 300, and may include, for example, a non-rechargeable primary battery, a rechargeable secondary battery, or a fuel battery. At least a part of the battery 370 may be disposed on substantially the same plane as, for example, the printed circuit board 380. The battery 370 may be integrally disposed inside the electronic device 200 or may be disposed in the electronic device 200 to be user detachable.

The first antenna 350 may be disposed between the display 220 and the support member 360. The first antenna 350 may include, for example, a near field communication (NFC) antenna, a wireless charging antenna, and/or a magnetic secure transmission (MST) antenna. The first antenna 350 may, for example, perform short-range communication with an external device, wirelessly transmit and receive power required for charging, and transmit short-range communication signals such as ones containing payment data. In another embodiment, an antenna structure may be formed by at least a portion of the side bezel structure 310 and/or a part of the support member 360 or a combination thereof.

The second antenna 355 may be disposed between the printed circuit board 380 and the rear plate 393. The second antenna 355 may include, for example, a near field communication (NFC) antenna, a wireless charging antenna, and/or a magnetic secure transmission (MST) antenna. For example, the second antenna 355 may perform short-range communication with an external device, wirelessly transmit and receive power required for charging, and may transmit a short-range communication signal such as ones containing payment data. In another embodiment, an antenna structure may be formed by at least a portion of the side bezel structure 310 and/or a part of the rear plate 393 or a combination thereof.

The sealing member 390 may be positioned between the side bezel structure 310 and the rear plate 393. The sealing member 390 may be configured to block moisture and foreign material flowing into the space surrounded by the side bezel structure 310 and the rear plate 393 from the outside.

According to an embodiment, a wearable device (e.g., the electronic device 200 illustrated in FIGS. 2A and 2B) may be worn and operated by a user (or a female). The wearable device may provide a women's health service by identifying (or obtaining) a body temperature of the user. The wearable device may be used to provide the women's health service.

According to an embodiment, the wearable device may be used to identify a climacteric symptom of a woman, and to guide early determination and early treatment of the climacteric symptom. For example, the wearable device may identify whether various symptoms occurring in climacteric occur to the user, and provide a guide with respect to the climacteric symptoms.

An operation of the wearable device according to the above-described embodiment may be described below. The wearable device described below may correspond to the electronic device 101 of FIG. 1 and/or the electronic device 200 of FIGS. 2A and 2B. The wearable electronic device may be implemented in various forms capable of being worn by the user, such as a smart watch, a smart band, a smart ring, a wireless earphone, or a smart glass.

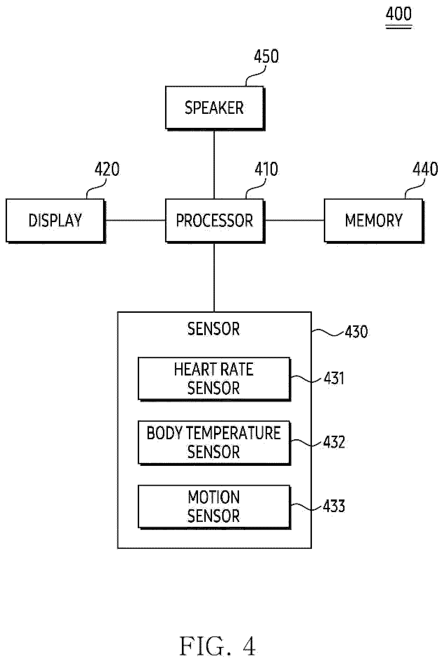

FIG. 4 is a simplified block diagram of a wearable device according to an embodiment.

Referring to FIG. 4, a wearable device 400 may correspond to the electronic device 101 of FIG. 1 and/or the electronic device 200 of FIGS. 2A and 2B. The wearable device 400 may include a processor 410, a display 420, a sensor 430, a memory 440, and/or a speaker 450. According to an embodiment, the wearable device 400 may include at least one of the processor 410, the display 420, the sensor 430, the memory 440, and the speaker 450. For example, at least a portion of the processor 410, the display 420, the sensor 430, the memory 440, and the speaker 450 may be omitted according to an embodiment.

According to an embodiment, the processor 410 may correspond to the processor 120 of FIG. 1. The processor 410 may be operatively (or operably) coupled with or connected with the display 420, the sensor 430, the memory 440, and the speaker 450. For example, the processor 410 being operably coupled with another component may mean that the processor 410 may control the other component. For example, the processor 410 may control the display 420, the sensor 430, the memory 440, and the speaker 450. display 420, the sensor 430, the memory 440, and the speaker 450 are controlled by the processor 410. For example, the processor 410 may determine an operation timing of the sensor 430. The processor 410 may control an operation of the sensor 430. The processor 410 may activate or inactive the sensor 430. The processor 410 may process information obtained from the sensor 430.

According to an embodiment, the processor 410 may be configured with at least one processor. The processor 120 may include at least one processor. For example, the processor 410 may include a main processor for processing requiring high performance and an auxiliary processor for processing requiring low power. For example, a body temperature sensor 432 may be connected to the auxiliary processor. As the body temperature sensor 432 is connected to the auxiliary processor, the body temperature sensor 432 may continuously monitor a body temperature of the user. According to an embodiment, a condition in which the main processor operates and a condition in which the auxiliary processor operates may be changed. For example, a processor (e.g., a main processor and an auxiliary processor) for processing the same data may be changed according to a situation.

According to an embodiment, the processor 410 may include a hardware component for processing data based on one or more instructions. The hardware component for processing data may include, for example, an arithmetic and logic unit (ALU), a field programmable gate array (FPGA), and/or a central processing unit (CPU).

According to an embodiment, the wearable device 400 may include the display 420. The display 420 may be used to display various screens. For example, the display 420 may be used to output content, data, or a signal through a screen. For example, the display 420 may display a screen processed by the processor 410. For example, the display 420 may be used to display information identified through the processor 410. For example, the display 420 may correspond to the display module 160 of FIG. 1.

According to an embodiment, the wearable device 400 may include the sensor 430. The sensor 430 may be used to obtain various external information. For example, the sensor 430 may be used to obtain data related to a body of the user. For example, the sensor 430 may be used to obtain body temperature data, heart rate data, and/or motion data of the user. For example, the sensor 430 may correspond to the sensor module 176 of FIG. 1.

According to an embodiment, the sensor 430 may be configured with a plurality of sensors. For example, the sensor 430 may include at least one of a heart rate sensor 431 (or a photoplethysmography (PPG) sensor), the body temperature sensor 432, and a motion sensor 433.

According to an embodiment, the heart rate sensor 431 may be used to measure a pulse (or a change of blood volume within a blood vessel) by identifying a change in an amount of photosensitivity of light according to a change in vascular volume. The processor 410 may identify a sleep state or a non-sleep state (or an active state) of the user, based on biometric data obtained through the heart rate sensor 431.

For example, the heart rate sensor 431 may include one or more photodiodes (PDs) and one or more light emitting diodes (LEDs). The LED may convert electrical energy into light energy. The PD may convert light energy into electrical energy. When light is transmitted from the LED to skin, the light may be partially absorbed by the skin, and at least a portion of remaining reflected light may be detected through the PD. For example, the LED may emit the light with one or more wavelengths. For example, the light emitted through the LED may include infrared radiation (IR) and visible light.

According to an embodiment, contact may be maintained for more than reference time in a state in which the heart rate sensor 431 (or the PPG sensor) including the PD and the LED is in contact with the skin. In a systolic phase of the heart of the user, since there is a lot of blood in blood vessels, an amount of light detected in the PD may decrease. In a diastolic phase of the heart of the user, since there is little blood in the blood vessels, the amount of light detected in the PD may increase. The heart rate sensor 431 may identify a DC signal obtained based on the skin and a vein, and an AC signal obtained based on an artery. The heart rate sensor 431 (or processor) may identify information on blood pressure, blood glucose, heart rate, and/or blood volume of the user, based on the obtained DC signal and the obtained AC signal.

According to an embodiment, the body temperature sensor 432 may be used to identify the body temperature (or a temperature of the skin) of the user and/or a temperature of an object. For example, the body temperature sensor 432 may include a non-contact infrared radiation (IR) temperature sensor or a contact temperature sensor.

For example, the contact temperature sensor may measure a temperature in a state of being in contact with a portion (or a measurement target) of the body of the user. The contact temperature sensor may include a thermocouple temperature sensor, a resistance temperature sensor, and a thermistor.

For example, the non-contact temperature sensor may measure a temperature in a state of being spaced apart from the portion of the body of the user. The non-contact temperature sensor may measure temperature based on the infrared emissivity of the portion (or the measurement target) of the body of the user. The non-contact temperature sensor may include an IR thermophile sensor (or a thermophile sensor).

For example, the portion (or the measurement target) of the body of the user may radiate (or emit) an electromagnetic wave of a wavelength corresponding to temperature. As the temperature increases, the wavelength of the radiated electromagnetic wave may decrease, and an amount of radiated energy may increase. A contact structure and an open structure may be formed inside the IR thermophile sensor. In case that a temperature difference occurs in the contact structure and the open structure, electromotive force may be generated by the seebeck effect according to the temperature difference. The IR thermophile sensor may identify the temperature based on the generated electromotive force.

For example, the skin temperature and the body temperature may be distinct from each other. The skin temperature may refer to a temperature of a skin surface at which the body temperature sensor 432 is located. The body temperature may refer to a temperature inside the human body cavity, such as the pulmonary artery, esophagus, bladder, external ear canal, or rectum. The body temperature sensor 432 may identify the body temperature of the user through a predefined algorithm (or learning technique), based on the measured skin temperature.

For example, the motion sensor 433 may be used to obtain data (e.g., value with respect to motion) related to motion of the wearable device 400 (or user). For example, the motion sensor 433 may include an acceleration sensor, a gyro sensor, a geomagnetic sensor, or a barometric pressure sensor. The acceleration sensor may identify (or measure or detect) acceleration of the wearable device 400 in three directions of an x-axis, a y-axis, and a z-axis. The gyro sensor may identify (or measure or detect) angular velocity of the wearable device 400 in the three directions of the x-axis, the y-axis, and the z-axis. The geomagnetic sensor may identify (or measure or detect) value with respect to an orientation by identifying geomagnetism. The barometric pressure sensor may identify (or measure or detect) barometric pressure around the wearable device 400.

Although not illustrated, the sensor 430 may further include various sensors for obtaining (or identifying, measuring, or detecting) various biometric data of the user.

For example, the sensor 430 may include a heart rate variability (HRV) sensor. The processor 410 may measure regularity or variability of a heart rate through the HRV sensor. The processor 410 may obtain information on the regularity or the variability of the heart rate through the HRV sensor.

For example, the sensor 430 may include an electrode sensor. The processor 410 may identify (or measure) electrodermal activity (EDA) through the electrode sensor. The processor 410 may identify information on a tension in the skin, based on the EDA.

For example, the sensor 430 may include a blood glucose sensor. The processor 410 may identify a blood glucose level of the user by identifying (or measuring) current generated by an electrochemical reaction with the blood glucose in blood.

According to an embodiment, the wearable device 400 may include the memory 440. The memory 440 may be used to store information or data. For example, the memory 440 may be used to store data obtained from the user. For example, the memory 440 may correspond to the memory 130 of FIG. 1. For example, the memory 440 may be a volatile memory unit or units. For example, the memory 440 may be a non-volatile memory unit or units. For example, the memory 440 may be a non-volatile memory unit or units. For example, the memory 440 may be another type of a computer-readable medium, such as a magnetic or optical disk. For example, the memory 440 may store data obtained based on an operation (e.g., algorithm execution operation) performed by the processor 410. For example, the memory 440 may store data (e.g., body temperature data) obtained from the sensor 430.

Although not illustrated, the wearable device 400 may further include communication circuitry. The communication circuitry may correspond to at least a portion of the communication module 190 of FIG. 1. For example, the communication circuitry may be used for various radio access technologies (RATs). For example, the communication circuitry may be used to perform Bluetooth communication or wireless local area network (WLAN) communication. For another example, the communication circuitry may be used to perform cellular communication. For example, the processor 410 may establish a connection with an external electronic device through the communication circuitry. For another example, the processor 410 may establish a connection with a server through the communication circuitry.

Although not illustrated, the wearable device 400 may further include an actuator. The actuator may correspond to the haptic module 179 of FIG. 1. For example, the actuator may be used to generate a vibration of the wearable device 400.

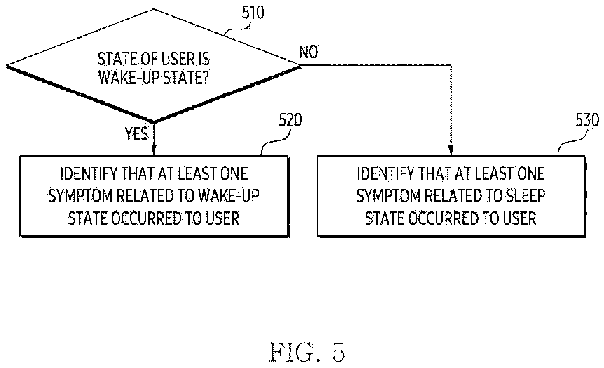

FIG. 5 is a flowchart illustrating an exemplary operation of a wearable device according to an embodiment.

According to an embodiment, operations 510 to 530 may be understood to be performed by a processor (e.g., the processor 410 of FIG. 4) of an electronic device (e.g., the wearable device 400 of FIG. 4).

Referring to FIG. 5, in operation 510, a processor 410 may identify whether a state of a user is a wake-up state. For example, the processor 410 may identify the state of the user as one of the wake-up state and a sleep state. For example, the processor 410 may identify whether the state of the user is the sleep state.

According to an embodiment, the processor 410 may identify whether the user is in the wake-up state using a heart rate sensor 431. For example, the processor 410 may obtain data related to a heart rate through the heart rate sensor 431. The processor 410 may identify whether the heart rate of the user is less than or equal to a designated range, based on the data related to the heart rate. The processor 410 may identify that the state of the user is not the wake-up state, based on identifying that the heart rate of the user is less than or equal to the designated range. The processor 410 may identify that the state of the user is the sleep state, based on identifying that the heart rate of the user is less than or equal to the designated range.

According to an embodiment, the processor 410 may identify whether the user is in the wake-up state, based on data identified through various sensors (e.g., a motion sensor 433 or an acceleration sensor), as well as the heart rate sensor 431. For example, the processor 410 may identify a value with respect to motion of the user through the motion sensor 433. The processor 410 may identify that the state of the user is not the wake-up state, based on identifying that the identified value with respect to the motion of the user is less than or equal to a reference value. The processor 410 may identify that the state of the user is the sleep state, based on identifying that the identified value with respect to the motion of the user is less than or equal to the reference value.

According to an embodiment, the processor 410 may identify whether the user is in the wake-up state, based on data identified through the heart rate sensor 431 and the motion sensor 433. The processor 410 may identify that the state of the user is the wake-up state, based on data related to the heart rate of the user identified using the heart rate sensor 431 and data related to the motion of the user identified using the motion sensor 433. The processor 410 may identify that the state of the user is the sleep state, based on the data related to the heart rate of the user identified using the heart rate sensor 431 and the data related to the motion of the user identified using the motion sensor 433. For example, the processor 410 may identify that the state of the user is the sleep state, based on identifying that the heart rate of the user is less than or equal to the designated range and that the value with respect to the motion of the user is less than or equal to the reference value. For example, the processor 410 may identify that the state of the user is the wake-up state, based on identifying that the heart rate of the user is outside the designated range and the value with respect to the motion of the user is greater than the reference value.

In operation 520, the processor 410 may identify that at least one symptom related to the wake-up state occurred to the user. For example, within a reference time interval (e.g., one month), the processor 410 may identify that the at least one symptom related to the wake-up state occurred to the user, based on the state of the user being the wake-up state. For example, in case that the state of the user is the wake-up state for a month, the processor 410 may identify that the at least one symptom related to the wake-up state occurred to the user. The processor 410 may identify whether the at least one symptom related to the wake-up state occurs to the user when the user is in the wake-up state for a month.

In operation 530, the processor 410 may identify that at least one symptom related to the sleep state occurred to the user. For example, the processor 410 may identify that the at least one symptom related to the sleep state occurred to the user within the reference time interval (e.g., a month) based on the state of the user being the sleep mode. For example, in case that the state of the user is the sleep state for a month, the processor 410 may identify that the at least one symptom related to the sleep state occurred to the user. The processor 410 may identify whether the at least one symptom related to the sleep state occurs to the user when the user is in the sleep state for a month.

According to an embodiment, various symptoms may appear in a climacteric female user. For example, in case that a state of the climacteric female user is the wake-up state, the climacteric female user may experience a sudden body temperature change symptom, a facial flushing symptom, a depression symptom, and/or an initial insomnia. For example, in case that the state of the climacteric female user is in the sleep state, a sleep fragmentation disorder symptom and/or an abnormal body temperature pattern symptom may occur to the climacteric female user.

According to an embodiment, a climacteric symptom may include at least one symptom related to the wake-up state and at least one symptom related to the sleep state. For example, the at least one symptom related to the wake-up state may refer to a symptom (or an identifiable symptom) identified in case that the state of the climacteric female user is the wake-up state. The at least one symptom related to the sleep state may mean a symptom (or an identifiable symptom) identified in case that the state of the climacteric female user is the sleep state.

Hereinafter, an embodiment for identifying various symptoms related to the climacteric symptom of the female user may be described. Operations for identifying various symptoms described below may be performed independently, and the operations may be used respectively, in order to identify one of symptoms related to various diseases other than a climacteric.

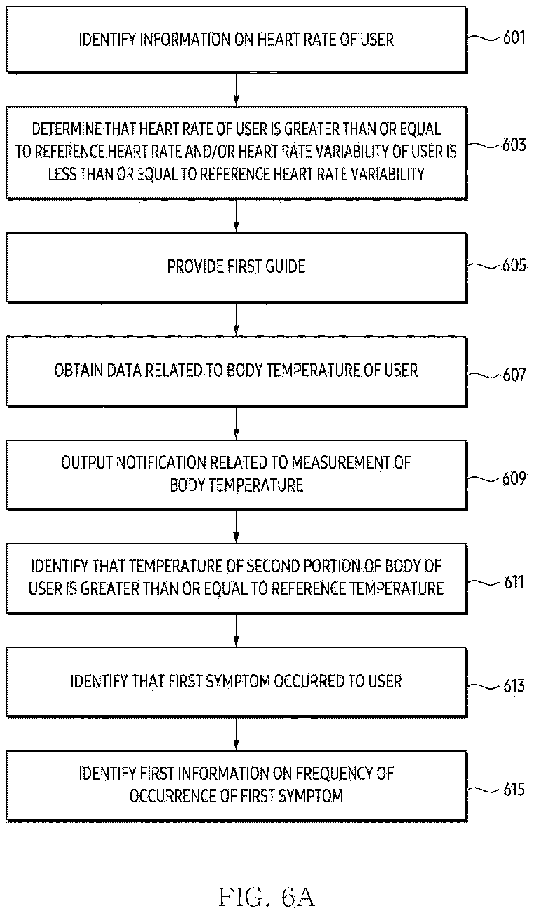

FIG. 6A is a flowchart illustrating an exemplary operation of a wearable device for identifying a first symptom according to an embodiment.

In the following embodiment, each operation may be performed sequentially, but is not necessarily performed sequentially. For example, the order of each operation may be changed, and at least two operations may be performed in parallel.

According to an embodiment, operations 601 to 615 may be understood to be performed by a processor (e.g., the processor 410 of FIG. 4) of an electronic device (e.g., the electronic device 400 of FIG. 4).

Referring to FIG. 6A, the operations 601 to 615 may be related to an operation of the processor 410 for identifying the first symptom of a user.

In operation 601, the processor 410 may identify information on a heart rate of the user. For example, the processor 410 may identify the information on the heart rate of the user, based on a signal obtained through a portion of a heart rate sensor 431 contacted with a first portion of a body of the user.

According to an embodiment, the heart rate sensor 431 may contact on the first portion of the body of the user. For example, the wearable device 400 may be worn on the first portion (e.g., wrist) of the body of the user. The heart rate sensor 431 may contact on the first portion of the body of the user.

According to an embodiment, the first symptom may include a facial flushing symptom. Before the facial flushing symptom appears in the user, a symptom in which a heart rate or heart rate variability increases may appear in the user. The processor 410 may monitor the heart rate or the heart rate variability of the user using the heart rate sensor 431.

In operation 603, the processor 410 may determine (or identify) that the heart rate of the user is greater than or equal to a reference heart rate and/or the heart rate variability of the user is less than or equal to reference heart rate variability. For example, the processor 410 may determine that the heart rate of the user is greater than or equal to the reference heart rate and/or the heart rate variability of the user is less than or equal to the reference heart rate variability, which are identified through the heart rate sensor 431 contacted on the first portion of the body of the user.

According to an embodiment, the processor 410 may determine that the heart rate of the user is greater than or equal to the reference heart rate by using the heart rate sensor 431. For example, the processor 410 may determine that the heart rate of the user is greater than or equal to the reference heart rate in order to identify a precursor symptom of the first symptom.

For example, the processor 410 may determine that the heart rate of the user is greater than or equal to the reference heart rate. Based on determining that the heart rate of the user is greater than or equal to the reference heart rate, the processor 410 may identify that the precursor symptom of the facial flushing symptom occurred to the user.

For example, the processor 410 may identify that the heart rate variability of the user is less than or equal to the reference heart rate variability. Based on determining that the heart rate variability of the user is less than or equal to the reference heart rate variability, the processor 410 may identify that the precursor symptom of the facial flushing symptom occurred to the user.

According to an embodiment, the processor 410 may identify information on a blood flow of the user by using a PPG sensor. For example, the processor 410 may determine that the blood flow of the user is greater than or equal to a reference blood flow. Based on determining that the blood flow of the user is greater than or equal to the reference blood flow, the processor 410 may identify that the precursor symptom of the facial flushing symptom occurred to the user.

In operation 605, the processor 410 may provide a first guide. For example, the processor 410 may provide the first guide proposing to contact a portion of a body temperature sensor on a second portion of the body of the user which is designated, based on determining that the heart rate of the user is greater than or equal to the reference heart rate and/or the heart rate variability of the user is less than or equal to the reference heart rate variability. For example, the processor 410 may provide the first guide proposing to contact the portion of the body temperature sensor on the second portion of the body of the user which is designated, based on determining that the blood flow of the user is greater than or equal to the reference blood flow by using the PPG sensor.

For example, the processor 410 may provide the first guide proposing to contact the wearable device 400 on the second portion (e.g., face) spaced apart from the first portion (e.g., wrist) of the body of the user.

For example, the processor 410 may display the first guide proposing to contact the wearable device 400 on the second portion of the body by using a display 420. For example, the processor 410 may display information on the heart rate of the user together with the first guide by using the display 420. For example, the processor 410 may display the first guide proposing to contact the wearable device 400 on the second portion of the body by using a display of an external electronic device (e.g., the electronic device 102 or 104 of FIG. 1) connected to the wearable device 400.

For example, the processor 410 may identify that the precursor symptom of the facial flushing symptom occurs in response to determining that the heart rate of the user is greater than or equal to the reference heart rate. The processor 410 may provide the first guide proposing to contact the wearable device 400 on the second portion of the body of the user spaced apart from the first portion of the body of the user, in order to identify whether the facial flushing symptom occurred.

For example, the processor 410 may identify that the precursor symptom of the facial flushing symptom occurs in response to determining that the heart rate variability of the user is less than or equal to the reference heart rate variability. The processor 410 may provide the first guide proposing to contact the wearable device 400 on the second portion of the body of the user spaced apart from the first portion of the body of the user, in order to identify whether the facial flushing symptom occurred.

According to an embodiment, based on determining that the heart rate of the user is greater than or equal to the reference heart rate, the processor 410 may identify that a value with respect to movement of the user is greater than or equal to a reference value by using a motion sensor 433. Based on identifying that the value with respect to the movement of the user is greater than or equal to the reference value, the processor 410 may refrain from providing the first guide. Based on identifying that the value with respect to the movement of the user is greater than or equal to the reference value, the processor 410 may not provide the first guide. For example, the processor 410 may identify whether an increase in the heart rate of the user to be greater than or equal to the reference heart rate, is due to the movement of the user. For example, in case that the value with respect to the movement of the user is greater than or equal to the reference value, the processor 410 may identify that the symptom in which the heart rate of the user is increased to be greater than or equal to the reference heart rate is not the precursor symptom of the facial flushing symptom. Even when the heart rate of the user is greater than or equal to the reference heart rate, the processor 410 may not provide the first guide in case that the value with respect to the movement of the user is greater than or equal to the reference value.

According to an embodiment, after the first guide is provided, the processor 410 may identify a user input to initiate obtaining of data related to a body temperature of the user in the second portion of the body of the user. For example, after the first guide is provided, the processor 410 may identify a user input for performing measurement of the facial flushing symptom.

According to an embodiment, the processor 410 may identify whether the wearable device 400 is spaced apart from the first portion of the body of the user by using a body temperature sensor 432. After the first guide is provided, the processor 410 may provide a second guide, based on identifying that the wearable device 400 maintains a state in contact with the first portion of the body of the user. For example, the processor 410 may additionally provide the second guide, based on identifying that the user does not release the wearable device 400 after the first guide is provided. For example, the processor 410 may provide the second guide for proposing to take off the wearable device 400. For example, the processor 410 may provide the second guide through a speaker 450. The second guide may include a notification notifying that the wearable device 400 will contact on the second portion spaced apart from the first portion. For example, the processor 410 may provide the second guide by outputting a beep sound through the speaker 450.

In operation 607, the processor 410 may obtain data related to the body temperature of the user. For example, after the first guide is provided, the processor 410 may obtain the data related to the body temperature of the user through the body temperature sensor 432. For example, after the first guide is provided, the processor 410 may obtain the data related to the body temperature of the user through the body temperature sensor 432 in response to identifying that the wearable device 400 contacts on the second portion spaced apart from the first portion of the body of the user through at least one of the body temperature sensor 432 and the motion sensor 433. For example, after the first guide is provided, the processor 410 may obtain the data related to the body temperature of the user through the body temperature sensor 432 in response to identifying that the wearable device 400 contacts on the second portion spaced apart from the first portion of the body of the user through a user input. For example, the user input may include a tap input, a long press input, and a double tap input on the display 420, a press input on a button, and an input on a button of the wearable device 400.

According to an embodiment, the processor 410 may change a state of the body temperature sensor 432 from an inactive state to an active state in response to identifying that the wearable device 400 contacts on the second portion of the body of the user. For example, the processor 410 may change (or switch) the state of the body temperature sensor 432 from the active state to the inactive state in response to identifying that the wearable device 400 is spaced apart from the first portion of the body of the user. After the state of the body temperature sensor 432 is changed to the inactive state, the processor 410 may change the state of the body temperature sensor 432 from the inactive state to the active state in response to identifying that the wearable device 400 contacts on the second portion of the body of the user. The processor 410 may obtain data related to the body temperature of the user through the body temperature sensor 432 changed to the active state.

According to an embodiment, the processor 410 may identify whether the wearable device 400 is in contact with the second portion of the body of the user after the wearable device 400 is spaced apart from the first portion of the body of the user. In case that the wearable device 400 is not in contact with the second portion of the body of the user within a designated time, the processor 410 may provide another notification for guiding the wearable device 400 to be in contacted with the second portion of the body of the user.

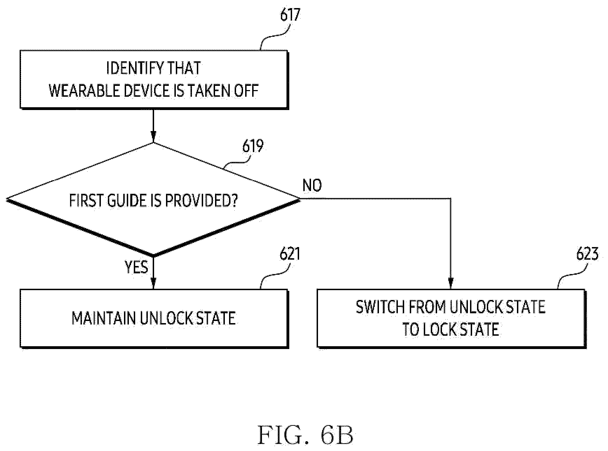

According to an embodiment, the processor 410 may maintain an unlock state even after the wearable device 400 is spaced apart from the first portion of the body of the user. The processor 410 may maintain a state of the wearable device 400 in the unlock state, based on identifying that the wearable device 400 is spaced apart from the first portion of the body of the user. An operation of the processor 410 for maintaining the state of the wearable device 400 in the unlock state will be described later in FIG. 6B.

In operation 609, the processor 410 may output a notification related to measurement of the body temperature. For example, the processor 410 may output the notification related to the measurement of the body temperature, based at least in part on obtaining data related to the body temperature. For example, based at least in part on obtaining the data related to the body temperature, the processor 410 may output the notification related to the measurement of the body temperature through the speaker 450.

According to an embodiment, the processor 410 may output the notification related to the measurement of the body temperature through the speaker 450 while obtaining the data related to the body temperature. After the data related to the body temperature is obtained, the processor 410 may cease outputting the notification related to the measurement of the body temperature, in order to guide the wearable device 400 to be spaced apart from the second portion of the body of the user. The processor 410 may store the obtained data related to the body temperature in a memory 440. For example, while measuring temperature of the second portion of the body of the user, the processor 410 may output the notification related to the measurement of the body temperature. The user of the wearable device 400 may not be able to see a display of the wearable device 400 while measuring the temperature of the second portion of the body of the user. The processor 410 may output the notification related to the measurement of the body temperature through the speaker 450 while obtaining the data related to the body temperature. After obtaining the data related to the body temperature, the processor 410 may change the state of the body temperature sensor 432 from the activated state to the inactive state.

According to an embodiment, the processor 410 may identify data related to the heart rate as well as the data related to the body temperature. For example, the processor 410 may identify the data related to the heart rate through the heart rate sensor 431 in response to identifying that the wearable device 400 is in contact with the second portion of the body of the user.

According to an embodiment, the processor 410 may obtain data related to the body temperature of the user by using an external electronic device connected to the wearable device 400. For example, the external electronic device may identify a first portion (e.g., a face) of the body of the user by using a camera of the external electronic device. The external electronic device may obtain an image related to the first portion of the body of the user. Based on a processing with respect to the obtained image, the external electronic device may obtain data related to a skin blood flow and/or data related to the body temperature in the first portion of the body of the user.

According to an embodiment, the processor 410 may provide at least one notification or at least one guide (e.g., the first guide or the second guide) by using the external electronic device connected to the wearable device 400. The processor 410 may provide the at least one notification or the at least one guide instead of the wearable device 400 by using the display of the wearable device 400 and/or the speaker of the wearable device 400. According to an embodiment, the processor 410 may receive a user input for selecting a device in which the at least one notification or the at least one guide is to be provided. Based on the received user input, the processor 410 may set the device in which the at least one notification or the at least one guide is to be provided as at least one of the wearable device 400 and the external electronic device.

For example, while obtaining the data related to the body temperature, the processor 410 may display a screen to guide maintaining a state that the wearable device 400 is contacted on the second portion of the body of the user by using a display of the external electronic device connected to the wearable device 400. The processor 410 may display the screen to guide maintaining the state that the wearable device 400 is contacted on the second portion of the body of the user, by controlling the external electronic device.

In operation 611, the processor 410 may identify that the temperature of the second portion of the body of the user is greater than or equal to a reference temperature. For example, the processor 410 may identify that the temperature of the second portion of the body of the user is greater than or equal to the reference temperature, based on the data related to the body temperature.

According to an embodiment, the processor 410 may change (or update) the reference temperature. For example, the processor 410 may identify a trend related to the body temperature of the user, based on the data related to the body temperature of the user. Based on the trend related to the body temperature of the user, the processor 410 may change (or update) the reference temperature.

According to an embodiment, the processor 410 may compare a trend related to the temperature of the second portion of the body of the user with the trend related to the body temperature of the user. The processor 410 may identify that a difference between the trend related to the temperature of the second portion of the body of the user and the trend related to the body temperature of the user is greater than or equal to a threshold range.

In operation 613, the processor 410 may identify that the first symptom occurred to the user. For example, the processor 410 may identify that the first symptom occurred to the user based on identifying that the temperature of the second portion of the body of the user is greater than or equal to the reference temperature. For example, based on identifying that the temperature of the face, which is the second portion of the body of the user, is greater than or equal to the reference temperature, the processor 410 may identify that the facial flushing symptom occurred to the user. For example, the processor 410 may identify that the first symptom among at least one symptom related to a wake-up state occurred to the user.

According to an embodiment, the processor 410 may identify that the first symptom occurred to the user, based on identifying that the difference between the trend related to the temperature of the second portion of the body of the user and the trend related to the body temperature of the user is greater than or equal to the threshold range.

In operation 615, the processor 410 may identify first information on a frequency of occurrence of the first symptom. For example, the processor 410 may identify the first information on the frequency of occurrence of the first symptom within a reference time interval (e.g., a month).

For example, the processor 410 may repeatedly perform the operations 601 to 613 within the reference time interval. The processor 410 may identify the number of times that the first symptom occurred (or appeared) within the reference time interval. Based on the number of times that the first symptom occurred, the processor 410 may identify the first information on the frequency of occurrence of the first symptom within the reference time interval. For example, the processor 410 may identify the frequency of occurrence of the first symptom as 90%, based on identifying that the first symptom has occurred for 27 days out of 30 days. For example, the processor 410 may identify whether the first symptom occurred to the user according to a designated time period. Based on the total number of measurements according to the designated time period, the processor 410 may identify a ratio of the number of times that the first symptom occurred as the frequency of occurrence of the first symptom.

FIG. 6B is a flowchart illustrating an exemplary operation of a wearable device for setting a lock state or an unlock state according to an embodiment.

In the following embodiment, each operation may be performed sequentially, but is not necessarily performed sequentially. For example, the order of each operation may be changed, and at least two operations may be performed in parallel.

According to an embodiment, operations 617 to 623 may be understood as being performed by a processor (e.g., the processor 410 of FIG. 4) of an electronic device (e.g., the electronic device 400 of FIG. 4).

In operation 617, the processor 410 may identify that a wearable device 400 is taken off. For example, using at least one of a heart rate sensor 431, a body temperature sensor 432, and a motion sensor 433, the processor 410 may identify that the wearable device 400 is taken off.

For example, using the heart rate sensor 431, the processor 410 may identify that the wearable device 400 is taken off. The processor 410 may identify that the wearable device 400 is taken off, based on identifying that a heart rate of a user is not detected.

For example, using the body temperature sensor 432, the processor 410 may identify that the wearable device 400 is taken off. The processor 410 may identify that the wearable device 400 is taken off, based on identifying that body temperature of the user is not detected.

For example, using the motion sensor 433, the processor 410 may identify that the wearable device 400 is taken off. The processor 410 may identify that a fixing portion (e.g., a buckle) of the wearable device 400 is released. After identifying that the fixing portion of the wearable device 400 is released, the processor 410 may identify that the wearable device 400 is taken off, based on a movement of the wearable device 400.

In operation 619, the processor 410 may identify whether a first guide is provided. For example, the processor 410 may identify whether the first guide is provided in response to identifying that the wearable device 400 is taken off.

According to an embodiment, the processor 410 may provide the first guide proposing to contact the wearable device 400 on a second portion (e.g., face) spaced apart from a first portion (e.g., wrist) of a body of the user. After the first guide is provided, the processor 410 may identify whether the wearable device 400 is taken off within a designated time.

In operation 621, in case that the first guide is provided, the processor 410 may maintain a state of the wearable device 400 in an unlock state. For example, based on identifying that the first guide is provided, the processor 410 may maintain the state of the wearable device 400 as the unlock state.

According to an embodiment, the state of the wearable device 400 may include a lock state and the unlock state. For example, in response to the wearable device 400 being worn by an authenticated user, the state of the wearable device 400 may be set to the unlock state. In response to identifying that the wearable device 400 is worn by the user, the processor 410 may identify body information (e.g., heart rate information or body temperature information) of the user. Based on the body information of the user, the processor 410 may identify whether the user wearing the wearable device 400 is the authenticated user. The processor 410 may set the state of the wearable device 400 to the unlock state. The processor 410 may switch the state of the wearable device 400 from the lock state to the unlock state. For example, while the wearable device 400 operates in the unlock state, the state of the wearable device 400 may be set to the lock state in response to the wearable device 400 being taken off. In response to the wearable device 400 being taken off, the processor 410 may switch the state of the wearable device 400 from the lock state to the unlock state.

According to an embodiment, the processor 410 may maintain the state of the wearable device 400 in the unlock state based on the first guide being provided. The processor 410 may maintain the state of the wearable device 400 in the unlock state based on identifying that the wearable device 400 is taken off within a designated time interval after the first guide is provided. For example, the processor 410 may set a lock waiting time. During the lock waiting time, the processor 410 may postpone changing the state of the wearable device 400 from the unlock state to the lock state. For example, based on the first guide being provided, the processor 410 may postpone changing the state of the wearable device 400 from the unlock state to the lock state during the lock waiting time. The processor 410 may identify whether a portion of the body temperature sensor 432 is in contact with the second portion of the body of the user within the lock waiting time. For example, based on the portion of the body temperature sensor 432 is in contact with the second portion of the body of the user, the processor 410 may maintain the state of the wearable device 400 in the unlock state. For example, the processor 410 may switch (or change) the state of the wearable device 400 from the unlock state to the lock state based on identifying that the portion of the body temperature sensor 432 is not in contact with the second portion of the body of the user and the lock waiting time elapses.

In operation 623, in case that the first guide is not provided, the processor 410 may switch the state of the wearable device 400 from the unlock state to the lock state. Based on the first guide not being provided, the processor 410 may switch the state of the wearable device 400 from the unlock state to the lock state.

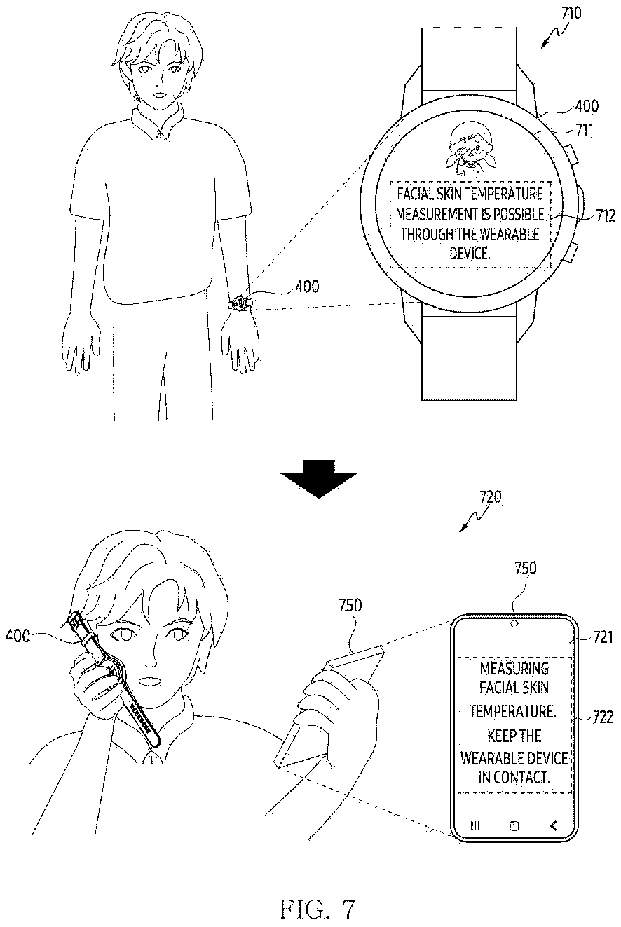

FIG. 7 illustrates an exemplary operation of a wearable device for identifying a first symptom according to an embodiment.

Referring to FIG. 7, in a state 710, a wearable device 400 may be in a state of being worn by a user. For example, the wearable device 400 may be in contact with a first portion (e.g., wrist) of the user. A processor 410 of the wearable device 400 may determine that a heart rate of the user is greater than or equal to a reference heart rate by using a heart rate sensor 431.

According to an embodiment, based on determining that the heart rate of the user is greater than or equal to the reference heart rate, the processor 410 may provide a first guide proposing to contact the wearable device 400 on a second portion (e.g., face) spaced apart from the first portion of the body of the user. For example, the processor 410 may display the first guide proposing to contact the wearable device 400 on the second portion spaced apart from the first portion of the user, through a screen 711 of the wearable device 400. The processor 410 may display text 712 related to the first guide within the screen 711. According to an embodiment, while displaying the screen 711, the processor 410 may provide the first guide with sound and/or vibration together.

According to an embodiment, the processor 410 may identify a user input for measuring a temperature of the second portion of the body of the user after the first guide is provided. Although not illustrated, the processor 410 may identify a user input for initiating an operation for measuring the temperature of the second portion of the body of the user from the user after the first guide is provided.

According to an embodiment, the processor 410 may identify whether the wearable device 400 is spaced apart from the first portion of the body of the user by using at least one of a body temperature sensor 432 and/or a motion sensor 433.

According to an embodiment, based on identifying that the wearable device 400 is spaced apart from the first portion of the body of the user, the processor 410 may identify that the wearable device 400 is taken off. In response to identifying that the wearable device 400 is taken off, the processor 410 may identify whether the first guide is provided. Based on the first guide being provided, the processor 410 may maintain the state of the wearable device 400 in an unlock state. After the first guide is provided, the processor 410 may maintain the state of the wearable device 400 in the unlocked state, based on identifying that the wearable device 400 is taken off within a designated time interval. For example, based on the first guide being provided, the processor 410 may postpone changing the state of the wearable device 400 from the unlock state to a lock state during a lock waiting time. For example, based on the first guide being provided, the processor 410 may refrain from changing the state of the wearable device 400 from the unlock state to the lock state during the lock waiting time.

In a state 720, the processor 410 may identify whether the wearable device 400 is in contacted with the second portion of the body of the user, by using at least one of the body temperature sensor 432 and/or the motion sensor 433. The processor 410 may identify that the wearable device 400 is contacted on the second portion of the body spaced apart from the first portion of the body of the user. In response to identifying that the wearable device 400 is contacted on the second portion of the body spaced apart from the first portion of the body of the user, the processor 410 may obtain data related to a body temperature of the user through the body temperature sensor 432.

According to an embodiment, based at least in part on obtaining the data related to the body temperature of the user, the processor 410 may provide a notification related to measurement of the body temperature. For example, based at least in part on obtaining the data related to the body temperature of the user, the processor 410 may output the notification related to the measurement of the body temperature. For example, based at least in part on obtaining the data related to the body temperature of the user, the processor 410 may output the notification related to the measurement of the body temperature.

According to an embodiment, the processor 410 may output (or provide) the notification related to the measurement of the body temperature by using a display of an external electronic device 750 (e.g., the electronic device 102 or 104 of FIG. 1) connected to the wearable device 400. For example, the processor 410 may display the notification related to the measurement of the body temperature of the user on a screen 721 by using the display of the external electronic device. The screen 721 may include text 722 indicating a measurement state of the body temperature of the user. For example, the processor 410 may display the screen 721 for maintaining the state in which the wearable device 400 is in contact with the second portion of the body of the user, by using the external electronic device 750.

According to an embodiment, the processor 410 may identify that a temperature of the second portion of the body of the user is greater than or equal to a reference temperature. Based on identifying that the temperature of the second portion of the body of the user is greater than or equal to the reference temperature, the processor 410 may identify that a first symptom occurred to the user. According to an embodiment, the processor 410 may identify a frequency of occurrence of the first symptom within a reference time interval (e.g., a month or three months). The processor 410 may identify first information on the frequency of occurrence of the first symptom within the reference time interval.

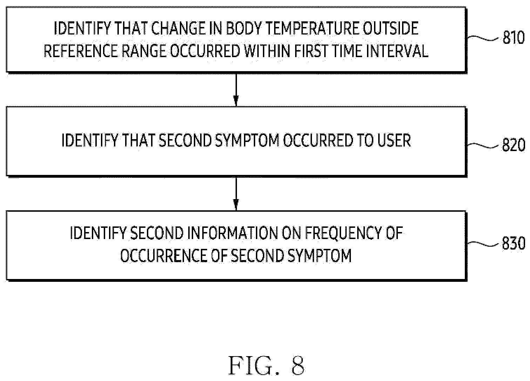

FIG. 8 is a flowchart illustrating an exemplary operation of a wearable device for identifying a second symptom according to an embodiment.

In the following embodiment, each operation may be performed sequentially, but is not necessarily performed sequentially. For example, the order of each operation may be changed, and at least two operations may be performed in parallel.

According to an embodiment, operations 810 to 830 may be understood to be performed by a processor (e.g., the processor 410 of FIG. 4) of an electronic device (e.g., the electronic device 400 of FIG. 4).

Referring to FIG. 8, in operation 810, the processor 410 may identify that a change in body temperature outside a reference range occurred within a first time interval. For example, the processor 410 may identify that the change in body temperature outside the reference range occurred within the first time interval in a state in which a wearable device 400 is in contact with a first portion of a user. For example, in case that a state of the user is a wake-up state, the processor 410 may identify that the change in body temperature outside the reference range occurred within the first time interval.

According to an embodiment, the processor 410 may identify that the change in body temperature outside the reference range (e.g., about 0.2 degrees) occurs within the first time interval (e.g., about 10 minutes). For example, the processor 410 may identify that the body temperature of the user is greater than or equal to a reference temperature (e.g., about 37.2 degrees) in a first timing. After identifying that the body temperature of the user is greater than or equal to the reference temperature, the processor 410 may identify that the body temperature of the user decreases in a second timing. The processor 410 may identify a time interval between the first timing and the second timing as the first time interval. The processor 410 may identify that an increase in body temperature and a decrease in body temperature occur repeatedly. According to an embodiment, the processor 410 may change (or update) the reference temperature. For example, the processor 410 may identify a trend related to the body temperature of the user based on data related to the body temperature of the user. Based on the trend related to the body temperature of the user, the processor 410 may change (or update) the reference temperature.

For example, the processor 410 may identify the body temperature of the user based on a first period (e.g., about 5 minutes). The processor 410 may identify that the body temperature of the user is greater than or equal to the reference temperature (e.g., about 37.2 degrees) in the first timing. The processor 410 may identify the body temperature of the user based on a second period (e.g., about 1 minute) from the first timing. The processor 410 may identify that the body temperature of the user decreases in the second timing. The processor 410 may identify that the body temperature of the user is a normal body temperature in a third timing after the second timing. The processor 410 may identify the body temperature of the user based on the first period from the third timing. For example, based on identifying that the body temperature of the user is greater than or equal to the reference temperature, the processor 410 may shortly set a time period measuring the body temperature of the user to the second period shorter than the first period. The processor 410 may increase the time period measuring the body temperature of the user from the second period to the first period, based on identifying that the body temperature of the user has returned to the normal body temperature.

According to an embodiment, the processor 410 may identify that the trend of the body temperature of the user is changed. The processor 410 may identify that a second trend of the body temperature of the user different from a stored first trend of the body temperature of the user is detected within the first time interval. The processor may identify that the change in body temperature outside the reference range occurred within the first time interval, based on a difference between the first trend and the second trend.

In operation 820, the processor 410 may identify that a second symptom occurred to the user. For example, based on identifying that a length of the first time interval is less than a length of a threshold time interval, the processor 410 may identify that the second symptom among at least one symptom related to the wake-up state occurred to the user.

According to an embodiment, the processor 410 may identify that the length of the first time interval is less than the length of the threshold time interval (e.g., about 10 minutes). For example, the processor 410 may identify that the length of the first time interval in which the change in body temperature outside the reference range occurred is less than the length of the threshold time interval. For example, the processor 410 may identify that a sudden change in body temperature has occurred to the user for a short time.

According to an embodiment, based on identifying that the length of the first time interval is less than the length of the threshold time interval, the processor 410 may identify that the second symptom occurred to the user. For example, based on identifying that the length of the first time interval is less than the length of the threshold time interval, the processor 410 may identify that the sudden change in body temperature occurred to the user during daily life.

According to an embodiment, the processor 410 may identify that a value with respect to a movement of the user is greater than or equal to a reference value by using a motion sensor 433, based on identifying that the change in body temperature outside the reference range occurred within the first time interval less than the length of the threshold time interval. The processor 410 may identify whether a cause in which the change in body temperature outside the reference range within the first time interval less than the length of the threshold time interval occurred is due to the movement of the user. Based on identifying that the value with respect to the movement of the user is greater than or equal to the reference value, the processor 410 may identify that the cause in which the change in body temperature outside the reference range within the first time interval less than the length of the threshold time interval occurred is due to the movement of the user. Based on identifying that the cause in which the change in body temperature occurred is due to the movement of the user, the processor 410 may identify that the second symptom not occurred to the user.

According to an embodiment, the processor 410 may identify that a location of the user is moved based on identifying that the change in body temperature outside the reference range occurred within the first time interval less than the length of the threshold time interval. For example, the processor 410 may identify the location of the user is moved by using a location-based service after identifying that the change in body temperature outside the reference range occurred within the first time interval less than the length of the threshold time interval.

For example, the processor 410 may identify that a location of the wearable device 400 is moved by using global positioning system (GPS) circuitry or communication circuitry, based on identifying that the change in body temperature outside the reference range occurred within the first time interval less than the length of the threshold time interval. The processor 410 may identify that the second symptom not occurred to the user, based on identifying that the cause in which the change in body temperature occurred is due to the movement of the location of the wearable device 400. For example, the processor 410 may identify that the user wearing the wearable device 400 entered a sauna by using at least one radio access technology (RAT). Even when the processor 410 identifies that the change in body temperature occurred to the user, the processor 410 may determine that the second symptom not occurred to the user.

According to an embodiment, the processor 410 may identify an external temperature of the wearable device 400, based on identifying that the change in body temperature outside the reference range occurred within the first time interval less than the length of the threshold time interval. The processor 410 may identify that an external temperature change occurred. The processor 410 may identify that the second symptom not occurred to the user, based on identifying that the cause in which the change in body temperature occurred is due to the change in external temperature.

In operation 830, the processor 410 may identify second information on a frequency of occurrence of the second symptom. For example, the processor 410 may identify the second information on the frequency of occurrence of the second symptom within a reference time interval (e.g., a month).

For example, the processor 410 may repeatedly perform the operations 810 and 820 within the reference time interval. The processor 410 may identify the number of times that the second symptom occurred (or appeared) within the reference time interval. The processor 410 may identify the second information on the frequency of occurrence of the second symptom within the reference time interval, based on the number of times that the second symptom occurred.

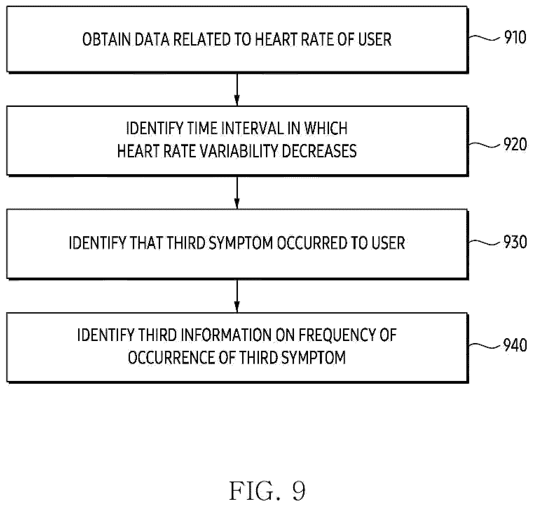

FIG. 9 is a flowchart illustrating an example of an operation of a wearable device for identifying a third symptom according to an embodiment.

In the following embodiment, each operation may be performed sequentially, but is not necessarily performed sequentially. For example, the order of each operation may be changed, and at least two operations may be performed in parallel.

According to an embodiment, operations 910 to 930 may be understood to be performed by a processor (e.g., the processor 410 of FIG. 4) of an electronic device (e.g., the electronic device 400 of FIG. 4).

Referring to FIG. 9, in operation 910, the processor 410 may obtain data related to a heart rate of a user. For example, the processor 410 may obtain the data related to the heart rate of the user by monitoring heart rate variability of the user by using a heart rate sensor 431. For example, the heart rate variability of the user may be identified based on a standard deviation between representative values (e.g., a maximum value, a minimum value, a median value, or an average value) of a PPG signal.

According to an embodiment, in case that a state of the user is a wake-up state for a designated duration (e.g., 30 days), the processor 410 may obtain the data related to the heart rate of the user. For example, the processor 410 may identify the heart rate variability of the user for the designated period. In case that the state of the user is the wake-up state, the processor 410 may identify representative (e.g., maximum, minimum, median, mode, or average) heart rate variability of the user.

In operation 920, the processor 410 may identify a time interval in which the heart rate variability of the user decreases. For example, in case that the state of the user is the wake-up state, the processor 410 may identify the time interval in which the heart rate variability of the user decreases. The processor 410 may identify the time interval in which the heart rate variability of the user decreases based on the data related to the heart rate of the user. For example, the processor 410 may identify that the heart rate variability of the current user decreased compared to representative heart rate variability of the user. The processor 410 may identify the time interval in which the heart rate variability of the user decreased. For example, the processor 410 may identify whether the decreased heart rate variability is maintained within the time interval.

In operation 930, the processor 410 may identify that a third symptom occurred to the user. For example, the processor 410 may identify that the third symptom occurred among at least one symptom related to the wake-up state to the user. For example, the processor 410 may identify at least one time interval in which the heart rate variability of the user decreases for a certain time (e.g., 1 day). The processor 410 may identify that the number of at least one time interval is greater than or equal to a threshold number. The processor 410 may identify that the third symptom occurred to the user, based on identifying that the number of at least one time interval is greater than or equal to the threshold number.

For example, the third symptom may include a depression symptom. The processor 410 may identify that the depression symptom occurred to the user, based on identifying that the number of at least one time interval in which the heart rate variability of the user decreases for the certain time (e.g., 1 day) is greater than or equal to the threshold number.

In operation 940, the processor 410 may identify third information on a frequency of occurrence of the third symptom. For example, the processor 410 may identify the third information on the frequency of occurrence of the third symptom within a reference time interval (e.g., a month).

For example, the processor 410 may repeatedly perform the operations 910 to 930 within the reference time interval. The processor 410 may identify the number of times that the third symptom occurred (or appeared) within the reference time interval. Based on the number of times that the third symptom occurred, the processor 410 may identify the third information on the frequency of occurrence of the third symptom within the reference time interval.

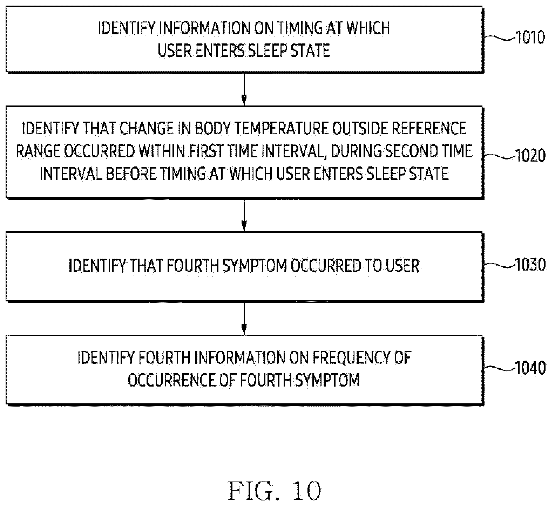

FIG. 10 is a flowchart illustrating an exemplary operation of a wearable device for identifying a fourth symptom according to an embodiment.

In the following embodiment, each operation may be performed sequentially, but is not necessarily performed sequentially. For example, the order of each operation may be changed, and at least two operations may be performed in parallel.

According to an embodiment, operations 1010 to 1040 may be understood as being performed by a processor (e.g., the processor 410 of FIG. 4) of an electronic device (e.g., the electronic device 400 of FIG. 4).

Referring to FIG. 10, in operation 1010, the processor 410 may identify information on a timing at which a user enters a sleep state. For example, the processor 410 may identify the information on the timing at which the user enters the sleep state, based on activity information of the user stored in a memory 340.

According to an embodiment, the processor 410 may store the activity information of the user in the memory 340. For example, the processor 410 may store a representative (e.g., maximum, minimum, median, mode, or average) sleep start time of the user, a representative (e.g., maximum, minimum, median, mode, or average) sleep end time of the user, and/or a representative (e.g., maximum, minimum, median, mode, or average) activity time of the user in the memory 340.

According to an embodiment, the processor 410 may identify information on a representative timing at which the user enters the sleep state based on the activity information of the user stored in the memory 340. For example, the processor 410 may identify the representative sleep start time of the user based on the activity information of the user.

According to an embodiment, the processor 410 may monitor a sleep time among the activity information of the user for a designated duration (e.g., 3 days). The processor 410 may update the representative sleep start time of the user by monitoring the sleep time of the user.

In operation 1020, the processor 410 may identify that a change in body temperature outside a reference range occurred within a first time interval during a second time interval before the timing at which the user enters the sleep state. For example, the processor 410 may identify that the change in body temperature outside the reference range occurred within an interval of about an hour in a state in which the wearable device 400 is in contact with a first portion of the user during the second time interval (e.g., about 2 hours) before the timing at which the user enters the sleep state.

For example, the processor 410 may identify that the body temperature of the user is greater than or equal to a reference temperature (e.g., about 37.2 degrees) in a first timing. After identifying that the body temperature of the user is greater than or equal to the reference temperature, the processor 410 may identify that the body temperature of the user decreases in a second timing. The processor 410 may identify a time interval between the first timing and the second timing as the first time interval. The processor 410 may identify that an increase in body temperature and a decrease in body temperature occur repeatedly. According to an embodiment, the processor 410 may change (or update) the reference temperature. For example, the processor 410 may identify a trend related to the body temperature of the user based on data related to the body temperature of the user. The processor 410 may change (or update) the reference temperature based on the trend related to the body temperature of the user.

For example, the processor 410 may identify the body temperature of the user based on a first period (e.g., about 5 minutes). The processor 410 may identify that the body temperature of the user is greater than or equal to the reference temperature (e.g., about 37.2 degrees) in the first timing. The processor 410 may identify the body temperature of the user based on a second period (e.g., about 1 minute) from the first timing. The processor 410 may identify that the body temperature of the user decreases in the second timing. The processor 410 may identify that the body temperature of the user is a normal body temperature in a third timing after the second timing. The processor 410 may identify the body temperature of the user based on the first period from the third timing. For example, based on identifying that the body temperature of the user is greater than or equal to the reference temperature, the processor 410 may shortly set a time period measuring the body temperature of the user to the second period shorter than the first period. The processor 410 may increase the time period measuring the body temperature of the user from the second period to the first period, based on identifying that the body temperature of the user has returned to the normal body temperature.

According to an embodiment, the processor 410 may identify that the trend of the body temperature of the user is changed. The processor 410 may identify that a second trend of the body temperature of the user different from a stored first trend of the body temperature of the user is detected within the first time interval. The processor may identify that the change in body temperature outside the reference range occurred within the first time interval, based on a difference between the first trend and the second trend.

In operation 1030, the processor 410 may identify that a fourth symptom occurred to the user. For example, the processor 410 may identify that the fourth symptom among at least one symptom related to the wake-up state occurred to the user, based on identifying that a length of the first time interval within the second time interval (e.g., about 2 hours) is less than a length of a threshold time interval (e.g., about 10 minutes). For example, the fourth symptom may be distinct from a second symptom. The second symptom may be a symptom in which a sudden change in body temperature of the user occurs rapidly during daily life. The fourth symptom may be a symptom in which a sleep disorder of the user occurs immediately before a sleep time. For example, the fourth symptom may include a symptom of insomnia due to a climacteric.

According to an embodiment, the processor 410 may identify that the length of the first time interval within the second time interval is less than the length of the threshold time interval (e.g., about 10 minutes). For example, the processor 410 may identify that the length of the first time interval in which the change in body temperature outside the reference range occurred is less than the length of the threshold time interval. For example, the processor 410 may identify that a sudden change in body temperature has occurred to the user for a short time within the second time interval before the timing at which the user enters the sleep state. The processor 410 may identify that the fourth symptom occurred to the user based on identifying that the sudden change in body temperature has occurred to the user for a short time within the second time interval before the timing at which the user enters the sleep state.

In operation 1040, the processor 410 may identify fourth information on a frequency of occurrence of the fourth symptom. For example, the processor 410 may identify the fourth information on the frequency of occurrence of the fourth symptom within a reference time interval (e.g., a month).

For example, the processor 410 may repeatedly perform the operations 1010 to 1030 within the reference time interval. The processor 410 may identify the number of times that the fourth symptom occurred (or appeared) within the reference time interval. Based on the number of times that the fourth symptom occurred, the processor 410 may identify the fourth information on the frequency of occurrence of the fourth symptom within the reference time interval.

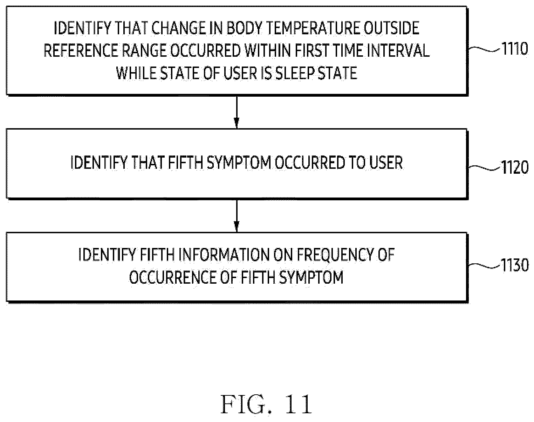

FIG. 11 is a flowchart illustrating an exemplary operation of a wearable device for identifying a fifth symptom according to an embodiment.

In the following embodiment, each operation may be performed sequentially, but is not necessarily performed sequentially. For example, the order of each operation may be changed, and at least two operations may be performed in parallel.

According to an embodiment, operations 1110 to 1130 may be understood as being performed by a processor (e.g., the processor 410 of FIG. 4) of an electronic device (e.g., the electronic device 400 of FIG. 4).

Referring to FIG. 11, in operation 1110, the processor 410 may identify that a change in body temperature outside a reference range occurred within a first time interval while a state of a user is a sleep state. For example, while the state of the user is the sleep state, the processor 410 may identify that the change in body temperature outside the reference range occurred within the first time interval in a state that a wearable device 400 is in contact with a first portion of a body of the user.

For example, the processor 410 may identify that the body temperature of the user is greater than or equal to a reference temperature (e.g., about 37.2 degrees) in a first timing within a time interval in which the state of the user is in the sleep state. After identifying that the body temperature of the user is greater than or equal to the reference temperature, the processor 410 may identify that the body temperature of the user decreases in a second timing within the time interval in which the state of the user is the sleep state. The processor 410 may identify a time interval between the first timing and the second timing as the first time interval. The processor 410 may identify that an increase in body temperature and a decrease in body temperature occur repeatedly.

For example, the processor 410 may identify the body temperature of the user based on a first period (e.g., about 5 minutes). The processor 410 may identify that the body temperature of the user is greater than or equal to the reference temperature (e.g., about 37.2 degrees) in the first timing. The processor 410 may identify the body temperature of the user based on a second period (e.g., about 1 minute) from the first timing. The processor 410 may identify that the body temperature of the user decreases in the second timing. The processor 410 may identify that the body temperature of the user is a normal body temperature in a third timing after the second timing. The processor 410 may identify the body temperature of the user based on the first period from the third timing. For example, based on identifying that the body temperature of the user is greater than or equal to the reference temperature, the processor 410 may shortly set a time period measuring the body temperature of the user to the second period shorter than the first period. The processor 410 may increase the time period measuring the body temperature of the user from the second period to the first period, based on identifying that the body temperature of the user has returned to the normal body temperature.

For example, operation 1110 may be related to the operation 810 of FIG. 8 or the operation 1020 of FIG. 10. The operation 810 of FIG. 8 may be an operation for identifying that the change in body temperature of the user occurred in case that the state of the user is the wake-up state. The operation 1020 of FIG. 10 may be an operation for identifying that the change in body temperature of the user occurred in case that the state of the user is a state before sleeping. The operation 1110 of FIG. 11 may be an operation for identifying that the change in body temperature of the user occurred in case that the state of the user is the sleep state.

In operation 1120, the processor 410 may identify that a fifth symptom occurred to the user. For example, the processor 410 may identify that the fifth symptom among at least one symptom related to the sleep state occurred to the user, based on identifying that a length of the first time interval is less than a length of a threshold time interval (e.g., about 10 minutes) while the state of the user is the sleep state. For example, the fifth symptom may include a sleep fragmentation symptom caused by a climacteric.

According to an embodiment, the processor 410 may identify that the length of the first time interval is less than the length of the threshold time interval (e.g., about 10 minutes) while the state of the user is the sleep state. For example, the processor 410 may identify that the length of the first time interval in which the change in body temperature outside the reference range occurred while the state of the user is in the sleep state is less than the length of the threshold time interval. For example, the processor 410 may identify that a sudden change in body temperature occurred to the user for a short time while the user was sleeping. The processor 410 may identify that the fifth symptom occurred to the user based on identifying that the sudden change in body temperature occurred to the user for the short time while the user was sleeping.

In operation 1130, the processor 410 may identify fifth information on a frequency of occurrence of the fifth symptom. For example, the processor 410 may identify the fifth information on the frequency of occurrence of the fifth symptom within a reference time interval (e.g., a month).

For example, the processor 410 may repeatedly perform the operations 1110 to 1120 within the reference time interval. The processor 410 may identify the number of times that the fifth symptom occurred (or appeared) within the reference time interval. Based on the number of times that the fifth symptom occurred, the processor 410 may identify the fifth information on the frequency of occurrence of the fifth symptom within the reference time interval.

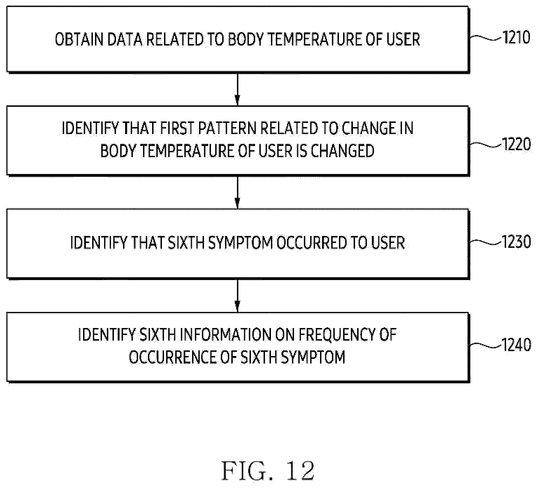

FIG. 12 is a flowchart illustrating an exemplary operation of a wearable device for identifying a sixth symptom according to an embodiment.

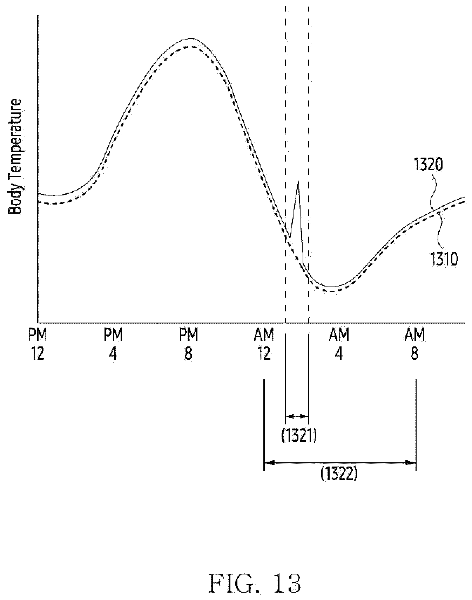

FIG. 13 illustrates an example of patterns related to a change in body temperature of a user according to an embodiment.

In the following embodiment, each operation may be performed sequentially, but is not necessarily performed sequentially. For example, the order of each operation may be changed, and at least two operations may be performed in parallel.

According to an embodiment, operations 1210 to 1240 may be understood as being performed by a processor (e.g., the processor 410 of FIG. 4) of an electronic device (e.g., the electronic device 400 of FIG. 4).

Referring to FIG. 12, in operation 1210, the processor 410 may obtain data related to a body temperature of a user. For example, the processor 410 may obtain the data related to the body temperature of the user while a state of the user is a sleep state.

According to an embodiment, the processor 410 may monitor a body temperature value of the user for a designated time (e.g., 3 days). Based on the monitored temperature value of the user, the processor 410 may identify a first pattern related to a change in the body temperature of the user. For example, the first pattern related to the change in body temperature of the user may indicate a change in body temperature of the user according to a circadian rhythm. For example, according to the first pattern, in case that the state is of the user is a wake-up state, the body temperature value of the user may be high. According to the first pattern, in case that the state of the user is the sleep state, the body temperature value of the user may be low. After the first pattern is identified, the processor 410 may identify data related to the body temperature of the user.

In operation 1220, the processor 410 may identify that the first pattern related to the change in body temperature of the user is changed. For example, in case that the state of the user is the sleep state, the processor 410 may identify that the first pattern related to the change in body temperature of the user is changed.

Referring to FIG. 13, a graph 1310 indicates the first pattern related to the change in body temperature of the user. A graph 1320 indicates a pattern related to the change in body temperature identified based on the data related to the body temperature of the user. The processor 410 may monitor the body temperature of the user. The processor 410 may identify an average change in body temperature of the user during a day, based on monitored information on the body temperature of the user. For example, the processor 410 may identify the average change in body temperature of the user during a day, based on the information on the body temperature of the user monitored for a designated time.

For example, the graph 1310 indicates the average change in body temperature of the user during a day. The graph 1320 indicates a newly identified change in body temperature of the user during a day. For example, an x-axis of the graph 1310 and the graph 1320 indicates a time during a day. A y-axis of the graph 1310 and the graph 1320 indicates a body temperature value of the user.

According to an embodiment, in an interval 1322, a state of the user may be a sleep state. For example, the processor 410 may identify that the state of the user is the sleep state in the interval 1322 based on the graph 1310. Based on the graph 1320, the processor 410 may identify that a sudden change in body temperature of the user occurred in an interval 1321. Based on identifying that the sudden change in body temperature of the user occurred, the processor 410 may identify that the first pattern related to the change in body temperature of the user is changed.

In operation 1230, the processor 410 may identify that a sixth symptom occurred to the user. For example, based on identifying that the first pattern related to the change in body temperature of the user is changed, the processor 410 may identify that the sixth symptom among at least one symptom related to the sleep state occurred to the user. For example, the sixth symptom may include a symptom in which an abnormal body temperature pattern occurs during sleep.

According to an embodiment, the processor 410 may identify the first pattern related to the average change in body temperature of the user during a day. The processor 410 may obtain data related to the body temperature of the user while the state of the user is the sleep state. The processor 410 may identify that the pattern related to the change in body temperature of the user identified based on the data related to the body temperature of the user is different from the first pattern. For example, the processor 410 may identify that the first pattern related to the change in body temperature of the user is changed. Based on identifying that the first pattern related to the change in body temperature of the user is changed, the processor 410 may identify that the sixth symptom occurred among the at least one symptom related to the sleep state to the user.

In operation 1240, the processor 410 may identify sixth information on a frequency of occurrence of the sixth symptom. For example, the processor 410 may identify the sixth information on the frequency of occurrence of the sixth symptom within a reference time interval (e.g., a month).

For example, the processor 410 may repeatedly perform the operations 1210 to 1230 within the reference time interval. The processor 410 may identify the number of times that the sixth symptom occurred (or appeared) within the reference time interval. Based on the number of times that the sixth symptom occurred, the processor 410 may identify the sixth information on the frequency of occurrence of the sixth symptom within the reference time interval.

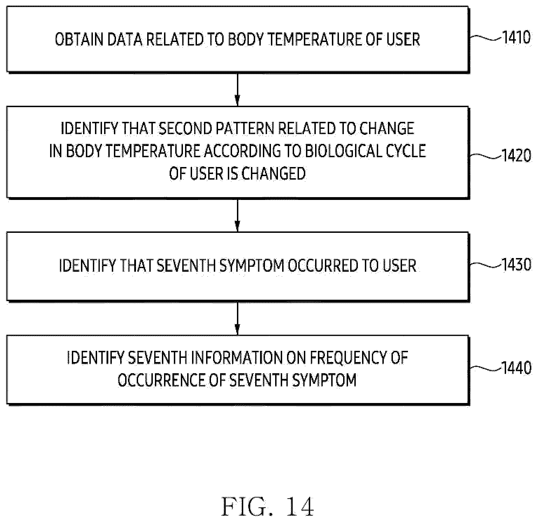

FIG. 14 is a flowchart illustrating an exemplary operation of a wearable device for identifying a seventh symptom according to an embodiment.

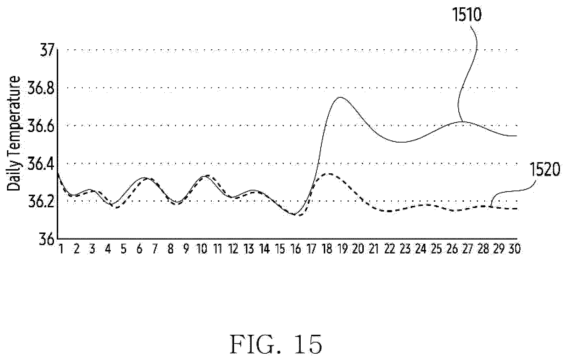

FIG. 15 illustrates an example of patterns related to a change in body temperature of a user according to an embodiment.

In the following embodiment, each operation may be performed sequentially, but is not necessarily performed sequentially. For example, the order of each operation may be changed, and at least two operations may be performed in parallel.

According to an embodiment, operations 1410 to 1440 may be understood as being performed by a processor (e.g., the processor 410 of FIG. 4) of an electronic device (e.g., the electronic device 400 of FIG. 4).

Referring to FIG. 14, in operation 1410, the processor 410 may obtain data related to a body temperature of a user. For example, the processor 410 may obtain the data related to the body temperature of the user while a state of the user is a sleep state.

According to an embodiment, the processor 410 may monitor a body temperature value of the user for a designated time (e.g., 30 days or a menstrual cycle). For example, the processor 410 may monitor the body temperature value of the user for the designated time in a state that the user is sleeping. The processor 410 may identify a second pattern related to a change in body temperature according to a biological cycle of the user, based on the monitored body temperature value of the user. For example, the second pattern related to the change in body temperature according to the biological cycle of the user may indicate a change in body temperature of the user according to the menstrual cycle. For example, the menstrual cycle may include a menstruation phase, a follicular phase, an ovulation phase, and a luteal phase. A body temperature of a female user may be changed based on the menstrual cycle. The processor 410 may identify the second pattern related to the change in body temperature according to the biological cycle of the user, based on the body temperature value of the user monitored for the designated time. For example, according to the second pattern, the body temperature (or basic body temperature) of the user may increase in the ovulation phase, and the body temperature of the user may gradually decrease in the luteal phase. After the second pattern is identified, the processor 410 may identify the data related to the body temperature of the user.

According to an embodiment, the processor 410 may identify the second pattern by receiving information on the biological cycle (e.g., menstrual cycle) of the user from the user. According to an embodiment, the processor 410 may obtain the information on the biological cycle of the user, based on data stored through an application (e.g., the application 146 of FIG. 1) for women's health.

In operation 1420, the processor 410 may identify that the second pattern related to the change in body temperature according to the biological cycle of the user is changed.

Referring to FIG. 15, a graph 1510 indicates the second pattern related to the change in body temperature according to the biological cycle of the user. A graph 1520 indicates a pattern related to the change in body temperature of the user identified based on the data related to the body temperature of the user. For example, the graph 1510 indicates an average change in body temperature of the user according to the biological cycle of the user. The graph 1520 indicates a newly identified change in body temperature of the user according to the biological cycle of the user. For example, an x-axis of the graph 1510 and the graph 1520 indicates days constituting the biological cycle of the user. A y-axis of the graph 1510 and the graph 1520 indicates a body temperature value (or a basic body temperature value) of the user. For example, according to the graph 1510, an interval including about 18 days may indicate the ovulation phase. After about 18 days have elapsed within a cycle of about 30 days, the body temperature of the user may increase. The processor 410 may identify that the second pattern related to the change in body temperature according to the biological cycle of the user is changed based on the graph 1520.

According to an embodiment, the processor 410 may identify that the graph 1520 indicating the pattern related to the change in body temperature of the user according to the biological cycle identified based on the data related to the body temperature of the user is distinct from the graph 1510 indicating the second pattern. The processor 410 may identify that the second pattern related to the change in body temperature according to the biological cycle of the user is changed.

Referring to FIG. 14, in operation 1430, the processor 410 may identify that a seventh symptom occurred to the user. For example, based on identifying that the second pattern related to the change in body temperature according to the biological cycle of the user is changed, the processor 410 may identify that the seventh symptom among at least one symptom related to the sleep state occurred to the user. For example, the seventh symptom may include a symptom in which the menstrual cycle is abnormally changed.

According to an embodiment, the processor 410 may identify the second pattern related to the average change in body temperature of the user during the menstrual cycle (e.g., about 30 days) of the user. The processor 410 may obtain the data related to the body temperature of the user during the menstrual cycle of the user. In case that the state of the user is the sleep state, the processor 410 may obtain the data related to the body temperature of the user. The processor 410 may obtain the data related to the body temperature of the user in the sleep state in which external factor do not significantly affect. The processor 410 may identify that the pattern related to the change in body temperature according to the biological cycle of the user identified based on the data related to the body temperature of the user is distinct from the second pattern. For example, the processor 410 may identify that the second pattern related to the change in body temperature according to the biological cycle of the user is changed. Based on identifying that the second pattern related to the change in body temperature according to the biological cycle of the user is changed, the processor 410 may identify that the seventh symptom occurred among the at least one symptom related to the sleep state to the user.

In operation 1440, the processor 410 may identify seventh information on a frequency of occurrence of the seventh symptom.

For example, the processor 410 may identify the seventh information on the frequency of occurrence of the seventh symptom within a reference time interval (e.g., a month).

For example, the processor 410 may identify whether the seventh symptom occurred within the reference time interval. The processor 410 may identify the number of times that the seventh symptom occurred (or appeared) within the reference time interval. The processor 410 may identify the seventh information on the frequency of occurrence of the seventh symptom within the reference time interval, based on the number of times that the seventh symptom occurred.

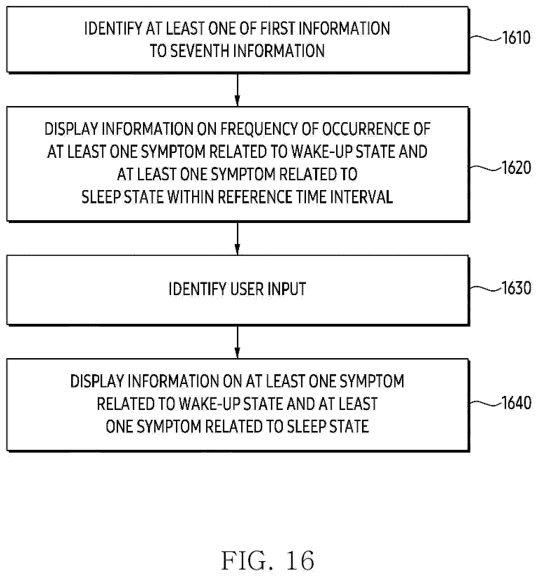

FIG. 16 is a flowchart illustrating an exemplary operation of a wearable device for displaying information on at least one symptom occurred to a user according to an embodiment.

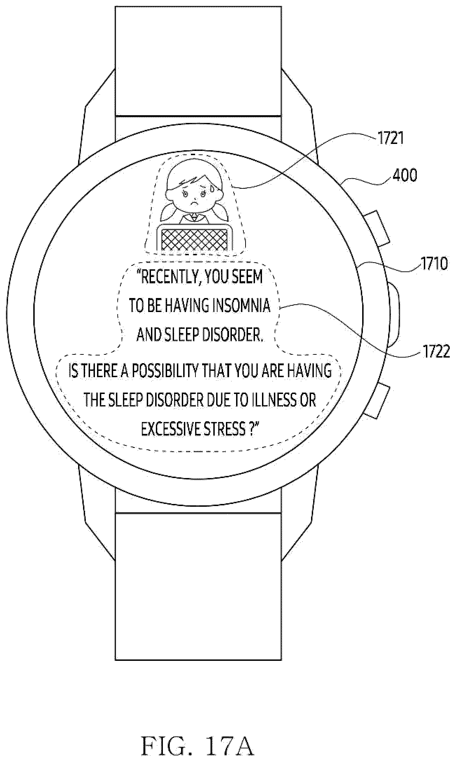

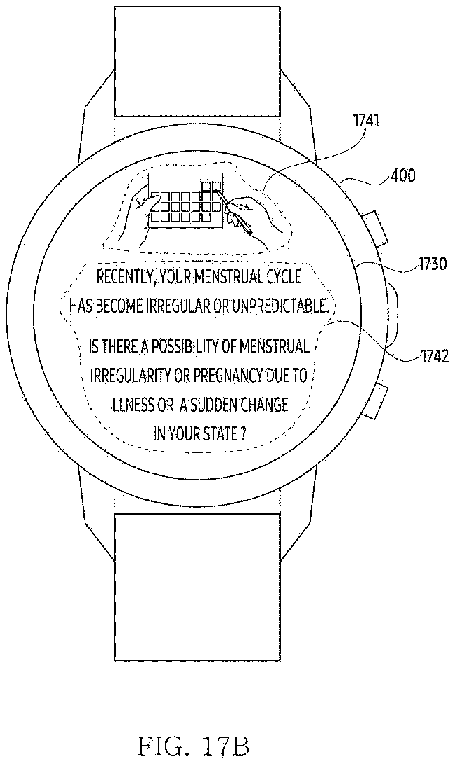

FIGS. 17A and 17B illustrate an example of an operation of an electronic device according to an embodiment.

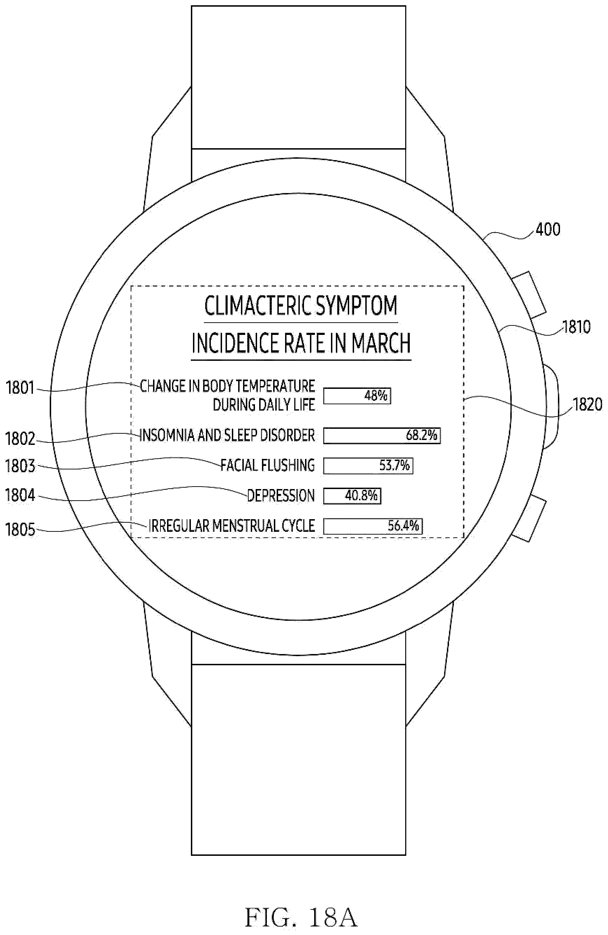

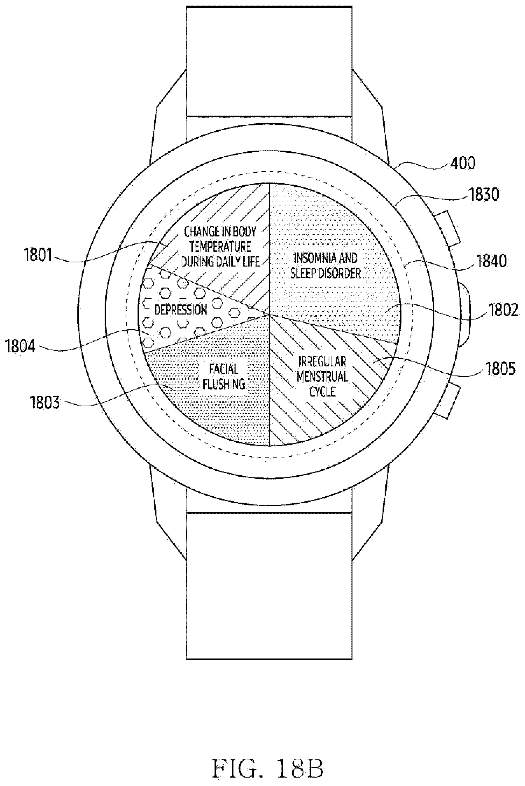

FIGS. 18A and 18B illustrate an example of an operation of an electronic device according to an embodiment.

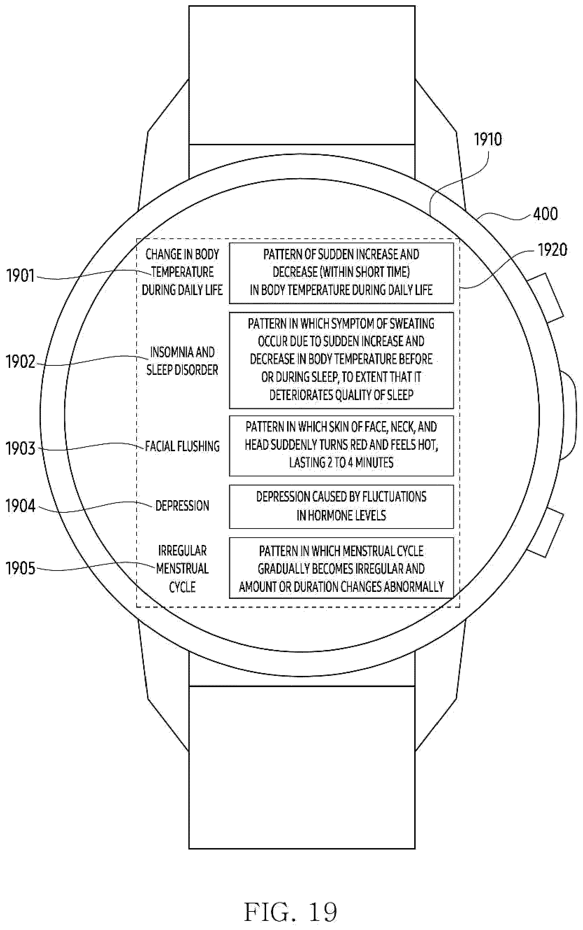

FIG. 19 illustrates an example of an operation of an electronic device according to an embodiment.

In the following embodiment, each operation may be performed sequentially, but is not necessarily performed sequentially. For example, the order of each operation may be changed, and at least two operations may be performed in parallel.

According to an embodiment, operations 1610 to 1640 may be understood to be performed by a processor (e.g., the processor 410 of FIG. 4) of an electronic device (e.g., the electronic device 400 of FIG. 4).

Referring to FIG. 16, in operation 1610, a processor 410 may identify at least one of first information to seventh information. For example, the processor 410 may identify the first information on a frequency of occurrence of a first symptom, based on the operations 610 to 670 of FIG. 6. For example, the processor 410 may identify a second information on a frequency of occurrence of a second symptom, based on the operations 810 to 830 of FIG. 8. For example, the processor 410 may identify a third information on a frequency of occurrence of a third symptom, based on the operations 910 to 940 of FIG. 9. For example, the processor 410 may identify a fourth information on a frequency of occurrence of a fourth symptom, based on the operations 1010 to 1040 of FIG. 10. For example, the processor 410 may identify a fifth information on a frequency of occurrence of a fifth symptom, based on the operations 1110 to 1130 of FIG. 11. For example, the processor 410 may identify a sixth information on a frequency of occurrence of a sixth symptom, based on the operations 1210 to 1240 of FIG. 12. For example, the processor 410 may identify the seventh information on a frequency of occurrence of a seventh symptom, based on the operations 1410 to 1440 of FIG. 14.

According to an embodiment, the processor 410 may receive an input for feedback of a user on at least one of the first information to the seventh information. The processor 410 may display a screen for receiving the input for the feedback of the user on the at least one of the first information to the seventh information on a display (e.g., the display 420 of FIG. 4). For example, the processor 410 may receive an input for feedback of the user on a cause of the first symptom, based on that the first symptom occurred to the user.

Referring to FIG. 17A, the processor 410 may display a screen 1710 for receiving an input for feedback of the user on the display 420, based on the fourth information and the fifth information. For example, the processor 410 may display text 1722 and/or an object 1721 for requesting the feedback of the user on a cause of the fourth symptom and the fifth symptom on the screen 1710, based on the fourth information and the fifth information. For example, the processor 410 may display the text 1722 and/or the object 1721 for requesting the feedback of the user on a cause of an insomnia symptom and/or a sleep fragmentation symptom on the screen 1710, based on the fourth information and the fifth information. For example, the processor 410 may display the text 1722 and/or the object 1721 with respect to a question to determine whether the cause of the insomnia symptom and/or the sleep fragmentation symptom is due to another disease or stress on the screen 1710. The processor 410 may receive the input for the feedback of the user based on information displayed on the screen 1710.

Referring to FIG. 17B, the processor 410 may display a screen 1730 for receiving an input for feedback of the user on the display 420, based on the seventh information. Based on the seventh information, the processor 410 may display text 1742 and/or an object 1741 for requesting the feedback of the user on a cause of the seventh symptom on the screen 1730. For example, based on the seventh information, the processor 410 may display the text 1742 and/or the object 1741 for requesting the feedback of the user on a cause of a symptom in which a menstrual cycle (or menstrual period) is abnormally changed on the screen 1730. For example, the processor 410 may display the text 1742 and/or the object 1741 with respect to a question to determine whether the cause of the symptom in which the menstrual cycle (or menstrual period) is abnormally changed is due to another disease or a sudden change in a state (e.g., weight loss, weight gain, or excessive stress) of the user on the screen 1730. The processor 410 may receive the input for the feedback of the user, based on information displayed on the screen 1730.

Referring to FIG. 16, in operation 1620, the processor 410 may display information on a frequency of occurrence of at least one symptom related to a wake-up state and at least one symptom related to a sleep state within a reference time interval. For example, the processor 410 may display the information on the frequency of occurrence of the at least one symptom related to the wake-up state and the at least one symptom related to the sleep state within the reference time interval by using the display 420.

According to an embodiment, the information on the frequency of occurrence of the at least one symptom related to the wake-up state and the at least one symptom related to the sleep state within the reference time interval may be displayed in various ways. For example, the processor 410 may identify a ratio with respect to days in which the at least one symptom related to the wake-up state and the at least one symptom related to the sleep state occurred, among a month (e.g., a 30 days), and may display the identified ratio. For example, the processor 410 may identify a relative ratio in which each symptom among all symptoms including the at least one symptom related to the wake-up state and the at least one symptom related to the sleep state takes up within a month, and display the identified ratio.

Referring to FIG. 18A, the processor 410 may display information on the frequency of occurrence of the at least one symptom related to the wake-up state and the at least one symptom related to the sleep state within the reference time interval on the display 420.

For example, the processor 410 may display the ratio with respect to days in which the at least one symptom related to the wake-up state and the at least one symptom related to the sleep state occurred, among a month (e.g., about 30 days).

For example, the processor 410 may identify whether the at least one symptom related to the wake-up state and the at least one symptom related to the sleep state occurred to the user according to a designated time interval. The processor 410 may identify a ratio of the number of times that the at least one symptom and the at least one symptom related to the sleep state occurred, based on the total number of measurements according to the designated time interval. The processor 410 may identify that a symptom 1801 to a symptom 1805 occurred to the user based on the designated time interval for a month. The processor 410 may display information on a ratio of the number of times that the symptom 1801 to the symptom 1805 occurred to the user on a screen 1810, based on the total number of measurements measured for a month.

For example, the processor 410 may identify that the symptom 1801 to the symptom 1805 occurred to the user for a month. The processor 410 may display information 1820 indicating a ratio with respect to days in which the symptom 1801 to the symptom 1805 occurred among a month by using a plurality of graphs. For example, the processor 410 may display information on a ratio in which the symptom 1801 occurred among a month on the screen 1810. The processor 410 may display information on a ratio in which a symptom 1802 occurred among a month on the screen 1810. The processor 410 may display information on a ratio in which a symptom 1803 occurred among a month on the screen 1810. The processor 410 may display information on a ratio in which a symptom 1804 occurred among a month on the screen 1810. The processor 410 may display information on a ratio in which the symptom 1805 occurred among a month on the screen 1810.

Referring to FIG. 18B, the processor 410 may display information on the frequency of occurrence of the at least one symptom related to the wake-up state and the at least one symptom related to the sleep state within the reference time interval on the display 420. For example, the processor 410 may display the relative ratio in which each symptom among all symptoms including the at least one symptom related to the wake-up state and the at least one symptom related to the sleep state takes up within a month on the display 420.

For example, the processor 410 may identify that the symptom 1801 to the symptom 1805 occurred to the user for a month. The processor 410 may display information 1840 indicating a ratio in which each of the symptom 1801 to the symptom 1805 takes up among a month on a screen 1830, by using a graph.

For example, the processor 410 may display information on a relative ratio in which the symptom 1801 occurred among all symptoms occurred to the user on the screen 1830. For example, the processor 410 may display information on a relative ratio in which the symptom 1802 occurred among all symptoms occurred to the user on the screen 1830. For example, the processor 410 may display information on a relative ratio in which the symptom 1803 occurred among all symptoms occurred to the user on the screen 1830. For example, the processor 410 may display information on a relative ratio in which the symptom 1804 occurred among all symptoms occurred to the user on the screen 1830. For example, the processor 410 may display information on a relative ratio in which the symptom 1805 occurred among all symptoms occurred to the user on the screen 1830.

Referring to FIG. 16, in operation 1630, the processor 410 may identify a user input. For example, the processor 410 may identify the user input while the information on the frequency of occurrence of the at least one symptom related to the wake-up state and the at least one symptom related to the sleep state within the reference time interval is displayed.

For example, the user input may include an input to a button of a wearable device 400, a tap input, a swipe input, a long press input, and a double tap input to a display of the wearable device 400. For example, the user input may include an input for displaying information on a symptom occurred to the user.

In operation 1640, the processor 410 may display the information on the at least one symptom related to the wake-up state and the at least one symptom related to the sleep state on the display 420. For example, the processor 410 may display the information on the at least one symptom related to the wake-up state and the at least one symptom related to the sleep state on the display 420 in response to the user input.

Referring to FIG. 19, the processor 410 may display information on the frequency of occurrence of the at least one symptom related to the wake-up state and the at least one symptom related to the sleep state within the reference time interval on a screen (e.g., the screen 1810 of FIG. 18A or the screen 1830 of FIG. 18B). While the screen is being displayed, the processor 410 may identify a user input. The processor 410 may display a screen 1910 in response to the user input. The processor 410 may display information 1920 on the at least one symptom related to the wake-up state and the at least one symptom related to the sleep state on the screen 1910. For example, the information 1920 may include information on a criterion for determining with respect to a symptom and/or a risk with respect to the symptom.

For example, the processor 410 may identify that a symptom 1901 to a symptom 1905 occurred to the user for one month. The processor 410 may display information on the symptom 1901 to the symptom 1905 on the screen 1910.

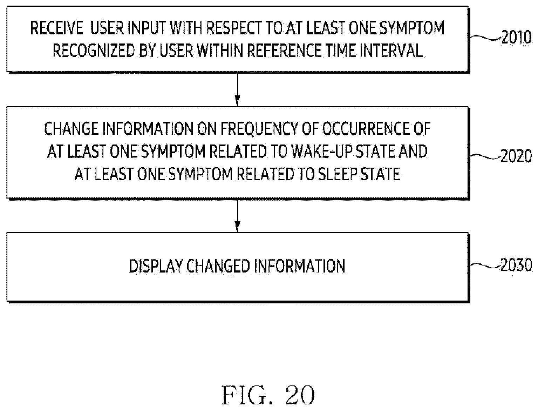

FIG. 20 is a flowchart illustrating an exemplary operation of a wearable device for displaying information on at least one symptom occurred to a user according to an embodiment.

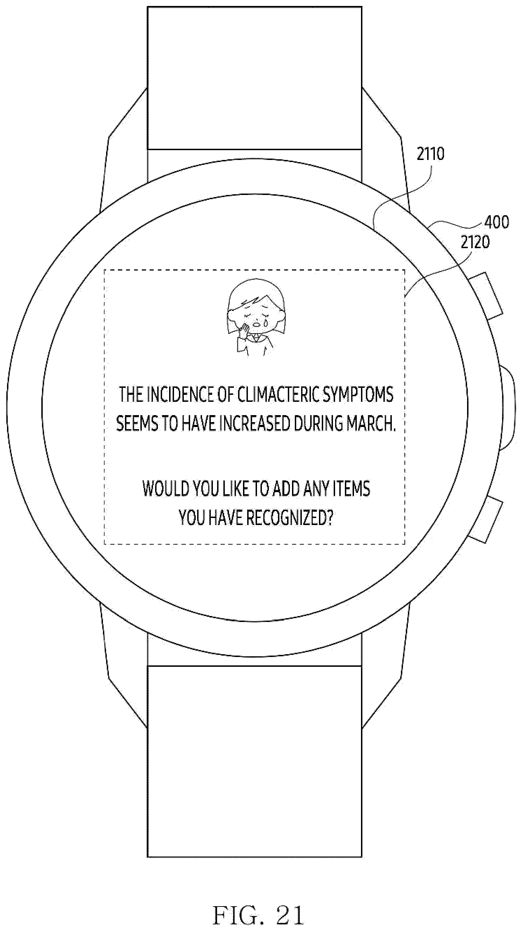

FIG. 21 illustrates an example of an operation of an electronic device according to an embodiment.

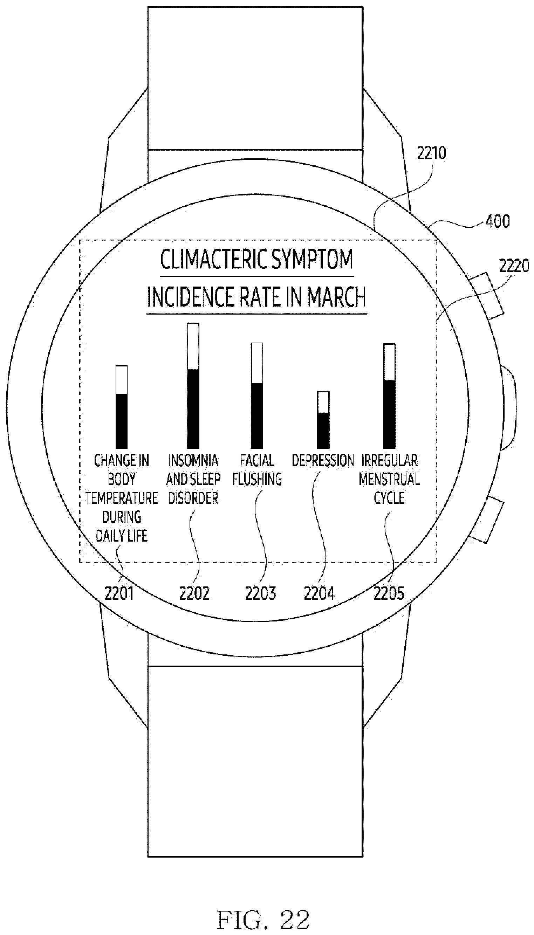

FIG. 22 illustrates an example of an operation of an electronic device according to an embodiment.

In the following embodiment, each operation may be performed sequentially, but is not necessarily performed sequentially. For example, the order of each operation may be changed, and at least two operations may be performed in parallel.

According to an embodiment, operations 2010 to 2030 may be understood to be performed by a processor (e.g., the processor 410 of FIG. 4) of an electronic device (e.g., the electronic device 400 of FIG. 4).

Referring to FIG. 20, in operation 2010, the processor 410 may receive a user input with respect to at least one symptom recognized by a user within a reference time interval. For example, the processor 410 may receive the user input with respect to the at least one symptom recognized by the user within the reference time interval after displaying information on a frequency of occurrence of at least one symptom related to a wake-up state and at least one symptom related to a sleep state within the reference time interval by using a display 420.

For example, the processor 410 may display the information on the frequency of occurrence of the at least one symptom related to the wake-up state and the at least one symptom related to the sleep state within the reference time interval, based on first information to seventh information. The processor 410 may receive the user input to change (or correct) the information on the frequency of occurrence of the at least one symptom related to the wake-up state and the at least one symptom related to the sleep state within the reference time interval.

According to an embodiment, the processor 410 may display a screen for receiving the user input with respect to the at least one symptom recognized by the user within the reference time interval.

Referring to FIG. 21, the processor 410 may display information 2120 for receiving the user input with respect to the at least one symptom recognized by the user within the reference time interval on a screen 2110. The processor 410 may receive the user input with respect to at least one symptom recognized by the user within the reference time interval after the information 2120 is displayed on the screen 2110.

Referring to FIG. 20, in operation 2020, the processor 410 may change the information on the frequency of occurrence of the at least one symptom related to the wake-up state and the at least one symptom related to the sleep state. For example, the processor 410 may change the information on the frequency of occurrence of the at least one symptom related to the wake-up state and the at least one symptom related to the sleep state, based on the user input. For example, the processor 410 may correct the information on the frequency of occurrence of the at least one symptom related to the wake-up state and the at least one symptom related to the sleep state within the reference time interval, based on the at least one symptom recognized by the user within the reference time interval.

According to an embodiment, the processor 410 may identify the frequency of occurrence of the at least one symptom related to the wake-up state and the at least one symptom related to the sleep state within the reference time interval, through a wearable device 400. For example, the identified frequency of occurrence may be different from frequency of occurrence of at least one symptom felt by an actual user. The processor 410 may change (or correct) the information on the frequency of occurrence of the at least one symptom related to the wake-up state and the at least one symptom related to the sleep state, based on the user input with respect to the at least one symptom recognized by the user within the reference time interval.

In operation 2030, the processor 410 may display changed information. For example, the processor 410 may display the changed information by using the display 420.

Referring to FIG. 22, the processor 410 may change (or correct) the information on the frequency of occurrence of the at least one symptom related to the wake-up state and the at least one symptom related to the sleep state, based on the user input with respect to the at least one symptom recognized by the user within the reference time interval. The processor 410 may display changed information 2220 on a screen 2210.

For example, the processor 410 may display the information 2220 on a frequency of occurrence of a symptom 2201 to a symptom 2205 by using at least one graph. At least one black bar graph among at least one graph illustrating information on the frequency of occurrence of the symptom 2201 to the symptom 2205 may indicate a frequency of occurrence identified through the wearable device 400. At least one white bar graph among at least one graph illustrating the information on the frequency of occurrence of the symptom 2201 to the symptom 2205 may indicate a frequency of occurrence with respect to the at least one symptom recognized by the user within the reference time interval.

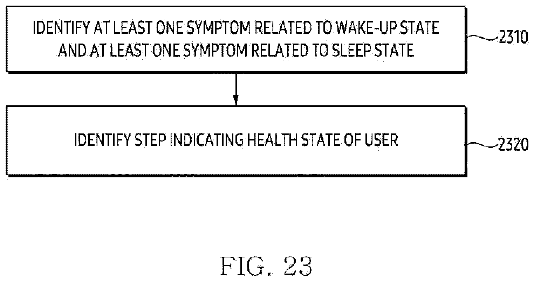

FIG. 23 is a flowchart illustrating an exemplary operation of a wearable device for identifying a step indicating a health state of a user according to an embodiment.

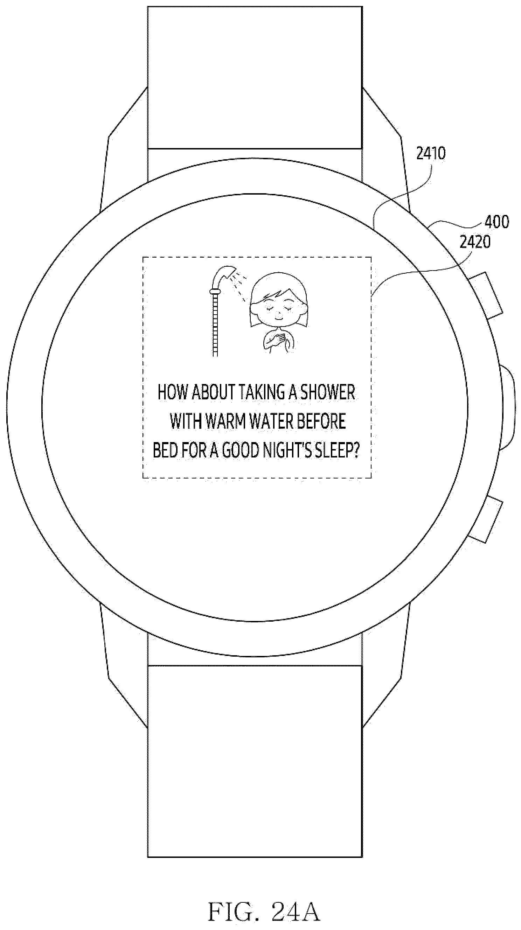

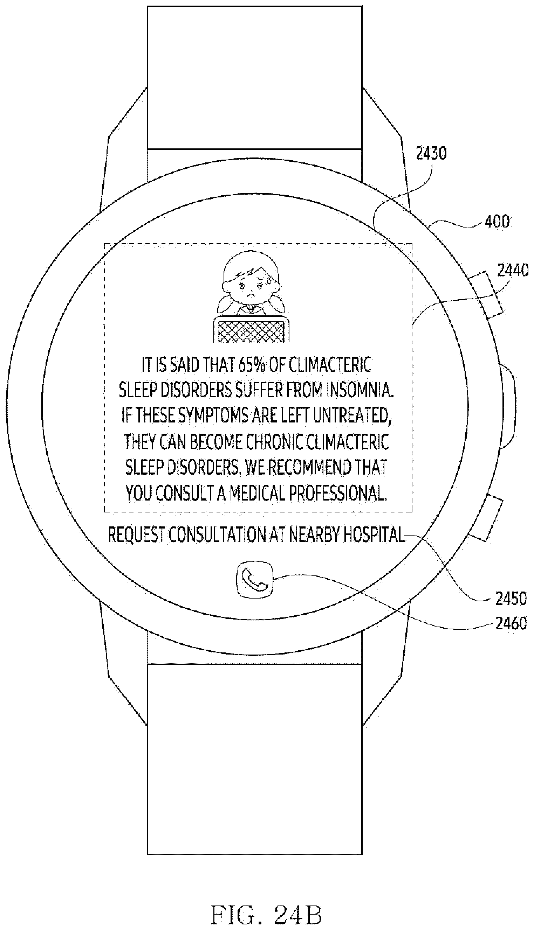

FIGS. 24A and 24B illustrate an example of an operation of an electronic device according to an embodiment.

In the following embodiment, each operation may be performed sequentially, but is not necessarily performed sequentially. For example, the order of each operation may be changed, and at least two operations may be performed in parallel.

According to an embodiment, operations 2310 to 2320 may be understood as being performed by a processor (e.g., the processor 410 of FIG. 4) of an electronic device (e.g., the electronic device 400 of FIG. 4).

Referring to FIG. 23, in operation 2310, a processor 410 may identify at least one symptom related to a wake-up state and at least one symptom related to a sleep state. For example, the processor 410 may identify the at least one symptom related to the wake-up state and the at least one symptom related to the sleep state, based on at least one of first information (e.g., the first information of FIG. 6) to seventh information (e.g., the seventh information of FIG. 14).

According to an embodiment, the processor 410 may identify body information (e.g., age, height, weight, dosage drug, and/or menstrual cycle) of a user along with the at least one symptom related to the wake-up state and the at least one symptom related to the sleep state. For example, the processor 410 may identify the body information of the user based on a user input.

In operation 2320, the processor 410 may identify a step indicating a health state of the user. For example, the processor 410 may identify the step indicating the health state of the user, based on the at least one symptom related to the wake-up state and the at least one symptom related to the sleep state. For example, based on the at least one symptom related to the wake-up state and the at least one symptom related to the sleep state, the processor 410 may identify the step indicating the health state of the user as one of a first step to a third step.

According to an embodiment, the processor 410 may identify the step indicating the health state of the user, based on the at least one symptom related to the wake-up state, the at least one symptom related to the sleep state, and/or the body information of the user.

According to an embodiment, the processor 410 may set at least one weight related to each of the at least one symptom related to the wake-up state, the at least one symptom related to the sleep state, and/or the body information of the user. The processor 410 may identify a value indicating the health state of the user by applying the at least one weight to a value for the at least one symptom related to the wake-up state, a value for the at least one symptom related to the sleep state, and a value for the body information of the user. The processor 410 may identify the step indicating the health state of the user, based on the value indicating the health state of the user.

For example, the processor 410 may set a first weight to a seventh weight to a frequency of occurrence of the first symptom to a frequency of occurrence of the seventh symptom, respectively. The processor 410 may set an eighth weight related to the body information of the user. The processor 410 may identify the value indicating the health state of the user, by applying the first to eighth weights to a value for the frequency of occurrence of the first symptom to a value for the frequency of occurrence of the seventh symptom, and the value indicating the body information of the user, respectively. The processor 410 may identify the step indicating the health state of the user, based on the value indicating the health state of the user.

According to an embodiment, the processor 410 may identify the step indicating the health state of the user as one of the first step to the third step, based on the value indicating the health state of the user. The processor 410 may identify the step indicating the health state of the user as the first step (e.g., a good step), based on the value indicating the health state of the user being greater than or equal to a first value and less than a second value. The processor 410 may identify the step indicating the health state of the user as a second step (e.g., an intermediate step), based on the value indicating the health state of the user being greater than or equal to the second value and less than a third value. The processor 410 may identify the step indicating the health state of the user as the third step (e.g., a serious step), based on the value indicating the health state of the user being greater than or equal to the third value.

According to an embodiment, the processor 410 may display information to guide at least one action related to the step indicating the health state of the user by using the display 420.

For example, the processor 410 may bypass an operation of displaying the information to guide the at least one action, based on the step indicating the health state of the user being the first step. The processor 410 may not display the information to guide the at least one action, based on the step indicating the health state of the user being the first step.

For example, the processor 410 may display the information to guide the at least one action, based on the step indicating the health state of the user being the second step.

For example, the processor 410 may display the information to guide the at least one action based on the step indicating the health state of the user being the third step. Based on the step indicating the health state of the user being the third step, the processor 410 may display a visual object for controlling an external electronic device connected to the wearable device 400, in order to perform at least one action together with the information to guide the at least one operation.

Referring to FIG. 24A, the processor 410 may display information 2420 to guide at least one action on a screen 2410, based on the step indicating the health state of the user being the second step. For example, the processor 410 may display text and/or an object to guide to take a shower with warm water on the screen 2410, based on the step indicating the health state of the user being the second step.

Referring to FIG. 24B, the processor 410 may display information 2440 to guide at least one action on a screen 2430, based on the step indicating the health state of the user being the third step. The processor 410 may display a visual object 2460 for controlling an external electronic device connected to the wearable device 400 on the screen 2430, in order to perform the at least one action together with the information 2440.

For example, the processor 410 may display text and/or an object to guide a consultation with a medical professional on the screen 2430, based on the step indicating the health state of the user being the third step. The processor 410 may display text 2450 proposing a consultation with a medical professional and a visual object 2460 for requesting a telephone consultation to the medical professional (or a hospital) with the information 2440 on the screen 2430. The processor 410 may control the external electronic device connected to the wearable device 400 in response to identifying a user input related to the visual object 2460. The processor 410 may perform a call connection to a medical professional through the external electronic device.

According to an embodiment, there is an effect of identifying a climacteric symptom based on information on a heart rate, information on a body temperature, and/or information on a movement of the user by using the wearable device 400. According to an embodiment, there is an effect of providing information indicating that climacteric symptom occurred to the user. According to an embodiment, there is an effect in capable of guiding the user in early medical care and treatment for the climacteric symptom.

According to an embodiment, the processor 410 may identify the at least one symptom related to the wake-up state and the at least one symptom related to the sleep state by performing the operations 1610 to 1640 of FIG. 16. The processor 410 may display the information on a frequency of occurrence of the at least one symptom related to the wake-up state and the at least one symptom related to the sleep state within a reference time interval on the display 420. The processor 410 may identify the step indicating the health state of the user, based on the information on the frequency of occurrence of the at least one symptom related to the wake-up state and the at least one symptom related to the sleep state within the reference time interval. The processor 410 may identify the step indicating the health state of the user as one of the first step, the second step, and the third step.

For example, the processor 410 may identify that the health state of the user is good based on identifying that the step indicating the health state of the user being the first step. Based on identifying that the step indicating the health state of the user being the first step, the processor 410 may identify that the user is in a good state, related to the climacteric symptom.

For example, the processor 410 may identify that the health state of the user is dangerous, based on identifying that the step indicating the health state of the user being the second step. Based on identifying that the step indicating the health state of the user being the second step, the processor 410 may identify that the user is in an intermediate state, related to the climacteric symptom.

For example, the processor 410 may identify that the health state of the user is serious, based on identifying that the step indicating the health state of the user being the third step. Based on identifying that the step indicating the health state of the user being the third step, the processor 410 may identify that the user is in a serious state, related to the climacteric symptom.

According to an embodiment, the processor 410 may monitor the step indicating the health state of the user. The processor 410 may display the screen 2410 of FIG. 24A or the screen 2430 of FIG. 24B, based on the monitored step indicating the health state of the user.

According to an embodiment, a wearable device may comprise a plurality of sensors including a heart rate sensor, a motion sensor, and a body temperature sensor, memory including a plurality of program instructions, and a processor operably coupled with the plurality of sensors and the memory. The program instructions, when executed by the processor, may cause the wearable device to identify, based on a signal obtained via a portion of the heart rate sensor contacted with a first portion of a body of a user, a heart rate of the user. The program instructions, when executed by the processor, may cause the wearable device to determine that the identified heart rate is greater than or equal to a reference heart rate. The program instructions, when executed by the processor, may cause the wearable device to, provide, in response to determining that the identified heart rate is greater than or equal to the reference heart rate, a first guide proposing to contact a portion of the body temperature sensor on a second portion of the body which is designated. The program instructions, when executed by the processor, may cause the wearable device to, after the first guide is provided, obtain, via the body temperature sensor, data related to a body temperature of the user. The program instructions, when executed by the processor, may cause the wearable device to output, based at least in part on obtaining the data related to the body temperature, notification related to measurement of the body temperature.

According to an embodiment, the program instructions, when executed by the processor, may cause the wearable device to determine, based on information on the heart rate of the user, that the heart rate of the user is greater than or equal to the reference heart rate, or heart rate variability of the user is less than or equal to reference heart rate variability. The program instructions, when executed by the processor, may cause the wearable device to provide, based on determining that the heart rate of the user is greater than or equal to the reference heart rate, or the heart rate variability of the user is less than or equal to the reference heart rate variability, the first guide.

According to an embodiment, the wearable device may comprise a display. The program instructions, when executed by the processor, may cause the wearable device to provide (or display), using the display, the first guide notifying to contact the wearable device on the second portion of the body, with the information on the heart rate of the user.

According to an embodiment, the program instructions, when executed by the processor, may cause the wearable device to, in response to identifying that the wearable device contacts on the second portion, change a state of the body temperature sensor from an inactive state to an active state. The program instructions, when executed by the processor, may cause the wearable device to obtain, via the body temperature sensor changed to the active state, data related to the body temperature of the user.

According to an embodiment, the program instructions, when executed by the processor, may cause the electronic device to, after the first guide is provided, provide a second guide proposing to take off the wearable device, based on identifying that the wearable device maintains a state of being in contacted with the first portion.

According to an embodiment, the wearable device may comprise a display. The program instructions, when executed by the processor, may cause the wearable device to display, using the display, the first guide notifying to contact the wearable device on the second portion of the body, with the information on the heart rate of the user.

According to an embodiment, the program instructions, when executed by the processor, may cause the wearable device to, in response to identifying that the wearable device contacts on the second portion, switch (or change) a state of the body temperature sensor from an inactive state to an active state, and identify, via the body temperature sensor switched (or changed) to the active state, data related to the body temperature of the user.

According to an embodiment, the program instructions, when executed by the processor, may cause the wearable device to, during identifying (or obtaining) the data related to the body temperature, provide (or output) the notification indicating a measurement state of the body temperature. The program instructions, when executed by the processor, may cause the wearable device to, after obtaining the data related to the body temperature, cease the output of the notification to instruct to space the wearable device apart from the second portion.

According to an embodiment, the program instructions, when executed by the processor, may cause the wearable device to, during identifying (or obtaining) the data related to the body temperature, display a screen to instruct maintaining a state that the wearable device is contacted on the second portion using an external electronic device, comprising a display, connected with the wearable device.

According to an embodiment, the program instructions, when executed by the processor, may cause the wearable device to check (or identify), based on the data related to the body temperature, that temperature of the second portion of the body is greater than or equal to a reference temperature. The program instructions, when executed by the processor, may cause the wearable device to, based on checking (or identifying) that the temperature of the second portion is greater than or equal to the reference temperature, check (or identify) that a first symptom occurred to the user. The program instructions, when executed by the processor, may cause the wearable device to identify first information on a frequency of occurrence of the first symptom within a reference time interval.

According to an embodiment, the wearable device may comprise a photoplethysmography (PPG) sensor for identifying information on a blood flow of the user. The program instructions, when executed by the processor, may cause the wearable device to identify, based on the data related to the body temperature, that temperature of the second portion is greater than or equal to the reference temperature or a blood flow of the second portion is greater than or equal to a reference blood flow. The program instructions, when executed by the processor, may cause the wearable device to, based on identifying that the temperature of the second portion is greater than or equal to the reference temperature or the blood flow of the second portion is greater than or equal to the reference blood flow, identify that the first symptom occurred to the user. The program instructions, when executed by the processor, may cause the wearable device to identify first information on a frequency of occurrence of the first symptom within a reference time interval.

According to an embodiment, the program instructions, when executed by the processor, may cause the wearable device to, within the reference time interval, based on a state of the user being a wake-up state, identify that at least one symptom related to the wake-up state occurred to the user. The program instructions, when executed by the processor, may cause the wearable device to, within the reference time interval, based on the state of the user being a sleep state, identify that at least one symptom related to the sleep state occurred to the user. The program instructions, when executed by the processor, may cause the wearable device to identify a step indicating a health state of the user, based on the at least one symptom related to the wake-up state and the at least one symptom related to the sleep state. The first symptom may be comprised in the at least one symptom related to the wake-up state.

According to an embodiment, the program instructions, when executed by the processor, may cause the wearable device to, in a state in which the wearable device is contacted with the first portion of the user, identify that a change in a body temperature outside a reference range occurred within a first time interval. The program instructions, when executed by the processor, may cause the wearable device to, based on identifying that a length of the first time interval is less than a length of a threshold time interval, identify that a second symptom among at least one symptom related to the wake-up state occurred to the user. The program instructions, when executed by the processor, may cause the wearable device to identify second information on a frequency of occurrence of the second symptom within the reference time interval.

According to an embodiment, the program instructions, when executed by the processor, may cause the wearable device to, based on activity information of the user stored in the memory, identify information on a timing at which the user enters the sleep state. The program instructions, when executed by the processor, may cause the wearable device to, during a second time interval before the timing at which the user enters the sleep state, identify the change in body temperature outside the reference range occurred within the first time interval. The program instructions, when executed by the processor, may cause the wearable device to, based on identifying that the length of the first time interval is less than the length of the threshold time interval, identify that a third symptom among the at least one symptom related to the sleep state, which is distinct from the second symptom, occurred to the user. The program instructions, when executed by the processor, may cause the wearable device to identify third information on a frequency of occurrence of the third symptom within the reference time interval.

According to an embodiment, the wearable device may comprise a display. The program instructions, when executed by the processor, may cause the wearable device to, based on at least one of the first information, the second information, and the third information, display, using the display, information on a frequency of occurrence of the at least one symptom related to the wake-up state and the at least one symptom related to the sleep state within the reference time interval.

According to an embodiment, the program instructions, when executed by the processor, may cause the wearable device to, while the information on the frequency of occurrence of the at least one symptom related to the wake-up state and the at least one symptom related to the sleep state within the reference time interval is displayed, identify a user input. The program instructions, when executed by the processor, may cause the wearable device to, in response to the user input, provide (or display), using the display, the information on the at least one symptom related to the wake-up state and the at least one symptom related to the sleep state.

According to an embodiment, the program instructions, when executed by the processor, may cause the wearable device to, after displaying, using the display, the information on the frequency of occurrence of the at least one symptom related to the wake-up state and the at least one symptom related to the sleep state within the reference time interval, receive a user input related to at least one symptom recognized by the user within the reference time interval. The program instructions, when executed by the processor, may cause the wearable device to change, based on the user input, the information on the frequency of occurrence of the at least one symptom related to the wake-up state and the at least one symptom related to the sleep state. The program instructions, when executed by the processor, may cause the wearable device to provide (or display) the changed information using the display.

According to an embodiment, the wearable device may further comprise a display, and wherein the program instructions, when executed by the processor, may cause the wearable device to, determine (or identify), based on the at least one symptom related to the wake-up state and the at least one symptom related to the sleep state, a step indicating a health state of the user as one of a first step to a third step. The program instructions, when executed by the processor, may cause the wearable device to, display, using the display, information for guiding at least one action related to the step indicating the health state of the user.

According to an embodiment, the program instructions, when executed by the processor, may cause the wearable device to, display, based on determining (or identifying) the step indicating the health state of the user as the third step, a visual object for controlling an external electronic device connected to the wearable device to perform the at least one action, with information for instructing the at least one action related to the third step.

According to an embodiment, a method of a wearable device may comprise identifying, based on a signal obtained via a portion of a heart rate sensor of the wearable device contacted with a first portion of a body of a user, a heart rate of the user. The method may comprise determining that the identified heart rate is greater than or equal to a reference heart rate. The method may comprise providing, in response to determining that the identified heart rate is greater than or equal to the reference heart rate, a first guide proposing to contact a portion of a body temperature sensor of the wearable device on a second portion of the body which is designated, and after the first guide is provided, obtaining, via the body temperature sensor, data related to a body temperature of the user. The method may comprise outputting, based at least in part on obtaining the data related to the body temperature, notification related to measurement of the body temperature.

According to an embodiment, providing the first guide proposing to contact the wearable device on the second portion may comprise displaying a screen for indicating the first guide proposing to contact the wearable device on the second portion of the body, with information on the heart rate of the user, using a display of the wearable device.

According to an embodiment, the method may comprise, based on the data related to the body temperature, identifying that temperature of the second portion is greater than or equal to a reference temperature. The method may comprise, based on identifying that temperature of the second portion is greater than or equal to the reference temperature, identifying that a first symptom occurred to the user. The method may comprise identifying first information on a frequency of occurrence of the first symptom within a reference time interval.

According to an embodiment, the method may comprise, within the reference time interval, based on a state of the user being a wake-up state, identifying that at least one symptom related to the wake-up state occurred to the user. The method may comprise, within the reference time interval, based on the state of the user being a sleep state, identifying that at least one symptom related to the sleep state occurred to the user. The method may comprise identifying a step indicating a health state of the user, based on the at least one symptom related to the wake-up state and the at least one symptom related to the sleep state. The first symptom may be comprised in the at least one symptom related to the wake-up state.

According to an embodiment, one or more programs stored in a non-transitory computer readable storage medium may comprise instructions which, when executed by a processor of a wearable device with a heart rate sensor, a body temperature sensor, and a motion sensor, cause the wearable device to identify, based on a signal obtained via a portion of the heart rate sensor contacted with a first portion of a body of a user, a heart rate of the user. The one or more programs, when executed by the processor of the wearable device, may comprise instructions that cause the wearable device to determine that the identified heart rate is greater than or equal to a reference heart rate. The one or more programs may comprise instructions that cause the wearable device to provide, in response to determining that the identified heart rate is greater than or equal to the reference heart rate, a first guide proposing to contact a portion of the body temperature sensor on a second portion of the body which is designated. The one or more programs may comprise instructions that cause the wearable device to, after the first guide is provided, obtain, via the body temperature sensor, data related to a body temperature of the user. The one or more programs may comprise instructions that cause the wearable device to output, based at least in part on obtaining the data related to the body temperature, notification related to measurement of the body temperature.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device comprising:
a plurality of sensors including a heart rate sensor, a motion sensor, and a body temperature sensor;
memory including a plurality of program instructions; and
a processor operably coupled with the plurality of sensors and the memory, wherein the program instructions, when executed by the processor, cause the wearable device to:
identify, based on a signal obtained via a portion of the heart rate sensor contacted with a first portion of a body of a user, information on a heart rate of the user,
provide, a first guide proposing to contact a portion of the body temperature sensor on a second portion of the body which is designated, based on the information on the heart rate of the user,
after the first guide is provided, obtain, via the body temperature sensor, data related to a body temperature of the user, and
output, based at least in part on obtaining the data related to the body temperature, notification related to measurement of the body temperature.

2. The wearable device of claim 1, wherein the program instructions, when executed by the processor, cause the wearable device to:
determine, based on the information on the heart rate of the user, that the heart rate of the user is greater than or equal to a reference heart rate or heart rate variability of the user is less than or equal to reference heart rate variability, and
provide, based on determining that the heart rate of the user is greater than or equal to the reference heart rate or the heart rate variability of the user is less than or equal to the reference heart rate variability, the first guide.

3. The wearable device of any one of claims 1 to 2, further comprising a display, and
wherein the program instructions, when executed by the processor, cause the wearable device to display, using the display, the first guide notifying to contact the wearable device on the second portion of the body, with the information on the heart rate of the user.

4. The wearable device of any one of claims 1 to 3, wherein the program instructions, when executed by the processor, cause the wearable device to:
in response to identifying that the wearable device contacts on the second portion, change a state of the body temperature sensor from an inactive state to an active state, and
obtain, via the body temperature sensor changed to the active state, data related to the body temperature of the user.

5. The wearable device of any one of claims 1 to 4, wherein the program instructions, when executed by the processor, cause the wearable device to:
during obtaining the data related to the body temperature, output the notification indicating a measurement state of the body temperature, and
after obtaining the data related to the body temperature, cease the output of the notification to guide to space the wearable device apart from the second portion.

6. The wearable device of claim 5, wherein the program instructions, when executed by the processor, cause the wearable device to, during obtaining the data related to the body temperature, display a screen to guide maintaining a state that the wearable device is contacted on the second portion using an external electronic device comprising a display connected with the wearable device.

7. The wearable device of any one of claims 1 to 6, further comprising a photoplethysmography (PPG) sensor for identifying information on a blood flow of the user, and
wherein the program instructions, when executed by the processor, cause the wearable device to:
identify, based on the data related to the body temperature, that a temperature of the second portion is greater than or equal to a reference temperature or a blood flow of the second portion is greater than or equal to a reference blood flow, and
based on identifying that the temperature of the second portion is greater than or equal to the reference temperature or the blood flow of the second portion is greater than or equal to the reference blood flow, identify that a first symptom occurred to the user, and
identify first information on a frequency of occurrence of the first symptom within a reference time interval.

8. The wearable device of claim 7, wherein the program instructions, when executed by the processor, cause the wearable device to:
within the reference time interval, based on a state of the user being a wake-up state, identify that at least one symptom related to the wake-up state occurred to the user,
within the reference time interval, based on the state of the user being a sleep state, identify that at least one symptom related to the sleep state occurred to the user, and
identify a step indicating a health state of the user, based on the at least one symptom related to the wake-up state and the at least one symptom related to the sleep state; and
wherein the first symptom is comprised in the at least one symptom related to the wake-up state.

9. The wearable device of claim 8, wherein the program instructions, when executed by the processor, cause the wearable device to:
in a state in which the wearable device is contacted with the first portion of the user, identify that a change in body temperature outside a reference range occurred within a first time interval,
based on identifying that a length of the first time interval is less than a length of a threshold time interval, identify that a second symptom among at least one symptom related to the wake-up state occurred to the user, and
identify second information on a frequency of occurrence of the second symptom within the reference time interval.

10. The wearable device of claim 9, wherein the program instructions, when executed by the processor, cause the wearable device to:
based on activity information of the user stored in the memory, identify information on a timing at which the user enters the sleep state,
during a second time interval before the timing at which the user enters the sleep state, identify the change in body temperature outside the reference range occurred within the first time interval,
based on identifying that the length of the first time interval is less than the length of the threshold time interval, identify that a third symptom among the at least one symptom related to the sleep state, which is distinct from the second symptom, occurred to the user, and
identify third information on a frequency of occurrence of the third symptom within the reference time interval.

11. The wearable device of claim 10 further comprising a display, and
wherein the program instructions, when executed by the processor, cause the wearable device to:
based on at least one of the first information, the second information, and the third information, display, using the display, information on a frequency of occurrence of the at least one symptom related to the wake-up state and the at least one symptom related to the sleep state within the reference time interval.

12. The wearable device of claim 11, wherein the program instructions, when executed by the processor, cause the wearable device to:
while the information on the frequency of occurrence of the at least one symptom related to the wake-up state and the at least one symptom related to the sleep state within the reference time interval is displayed, identify a user input, and
in response to the user input, display, using the display, the information on the at least one symptom related to the wake-up state and the at least one symptom related to the sleep state.

13. The wearable device of any one of claims 11 to 12, wherein the processor is further configured to:
after displaying, using the display, the information on the frequency of occurrence of the at least one symptom related to the wake-up state and the at least one symptom related to the sleep state within the reference time interval, receive a user input related to at least one symptom recognized by the user within the reference time interval, and
change, based on the user input, the information on the frequency of occurrence of the at least one symptom related to the wake-up state and the at least one symptom related to the sleep state, and
display the changed information using the display.

14. A method of a wearable device, comprising:
identifying, based on a signal obtained via a portion of a heart rate sensor of the wearable device contacted with a first portion of a body of a user, a heart rate of the user,
determining that the identified heart rate is greater than or equal to a reference heart rate,
providing, in response to determining that the identified heart rate is greater than or equal to the reference heart rate, a first guide proposing to contact a portion of a body temperature sensor of the wearable device with a second portion of the body which is designated,
after the first guide is provided, obtaining, via the body temperature sensor, data related to a body temperature of the user, and
outputting, based at least in part on obtaining the data related to the body temperature, notification related to measurement of the body temperature.

15. A non-transitory computer readable storage medium storing one or more programs, wherein the one or more programs comprise instructions which, when executed by a processor of a wearable device with a heart rate sensor, a body temperature sensor, and a motion sensor, cause the wearable device to:
identify, based on a signal obtained via a portion of the heart rate sensor contacted with a first portion of a body of a user, information on a heart rate of the user,
provide, based on the information on the heart rate of the user, a first guide proposing to contact a portion of the body temperature sensor on a second portion of the body which is designated,
after the first guide is provided, obtain, via the body temperature sensor, data related to a body temperature of the user, and
output, based at least in part on obtaining the data related to the body temperature, notification related to measurement of the body temperature.
